# FASCICULE DE BREVET EUROPEEN

(11) **EP 0 596 812 B1**
(45) Date de publication et mention de la délivrance du brevet: **01.10.1997**
(21) Numéro de dépôt: 93420334.0
(22) Date de dépôt: 06.08.1993
(51) Int. Cl.: C12N 15/55, C12N 9/78, C12N 1/21, C12P 7/40, C12P 7/44

(54) **Nitrilase recombinante et son utilisation**
Rekombinantes Nitrilase und deren Verwendung
Recombinant nitrilase and use thereof

(30) Priorité: 10.08.1992 FR 9209882
(43) Date de publication de la demande: 11.05.1994
(73) Titulaire: RHONE-POULENC NUTRITION ANIMALE, 92160 Antony (FR)
(72) Inventeur: Petre, Dominique, F-69006 Lyon (FR); Cerbelaud, Edith, F-69350 La Mulatiere (FR); Levy-Schil, Sophie, F-75007 Paris (FR); Crouzet, Joel, F-75012 Paris (FR)
(74) Mandataire: Ropital-Bonvarlet, Claude

(56) Documents cités:
- EP-A- 0 412 465
- WO-A-89/00193
- US-A- 4 629 700

## Description

La présente invention a pour objet de nouveaux polypeptides présentant une activité nitrilase, les outils de génie génétique pour les produire, à savoir une séquence d'ADN, les cassettes d'expression portant cette séquence d'ADN recombinant et les micro-organismes recombinés (micro-organismes hôtes) contenant ladite séquence d'ADN.

La présente invention a également pour objet un procédé de transformation enzymatique des nitriles en carboxylates au moyen des polypeptides selon l'invention ou d'un micro-organisme hôte contenant la séquence d'ADN selon l'invention. Une application particulière du procédé de l'invention est la synthèse enzymatique de l'adipate d'ammonium ou du cyano-5 valérate d'ammonium par hydrolyse de l'adiponitrile à l'aide d'un polypeptide ou d'un micro-organisme hôte selon l'invention.

On sait que l'adipate d'ammonium est un produit particulièrement intéressant car il peut être transformé en acide adipique, un produit lui-même largement utilisé pour la préparation du Nylon-6,6.

L'hydrolyse enzymatique des dinitriles a été décrite par de nombreux auteurs. Cependant, les voies d'hydrolyse de ces dinitriles en acides organiques ne sont pas souvent évoquées. Le schéma théorique d'hydrolyse est le suivant : 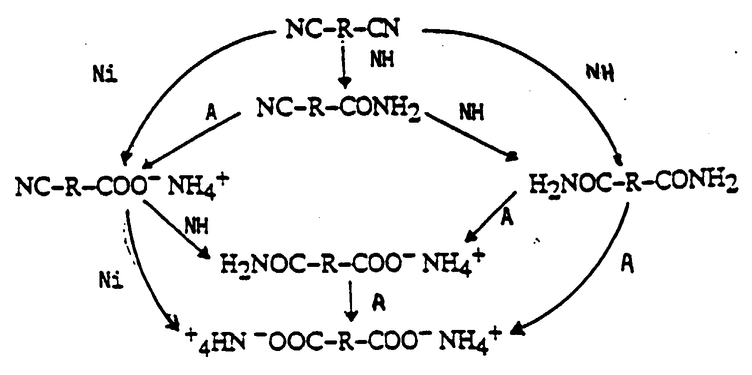
- NH =: nitrile hydratase
- Ni =: nitrilase
- A =: amidase
- R =: (CH₂)ₙ, n étant un nombre entier égal à 4 dans le cas de l'adiponitrile.

En fait, on note très souvent que certaines voies sont privilégiées et que certains produits ne se forment pas ou bien ne sont pas hydrolysés.

Parmi les micro-organismes chez lesquels on a pu démontrer l'existence d'une activité enzymatique permettant cette hydrolyse, on peut citer notamment les souches appartenant au genre *Fusarium,* qui dégradent le succinonitrile et l'adiponitrile sans indication sur les produits de la réaction [Goldlust et al., Biotechnol. and Appl. Biochem., 1989, 11, 581] ; les souches appartenant au genre *Pseudomonas,* qui dégradent l'adiponitrile [Yanase et al., Agric. Biol. Chem., 1982, 46, 2925] ; et les souches appartenant au genre *Rhodococcus,* en particulier *Rhodococcus rhodochrous* NCIB 11 216, qui hydrolyse l'adiponitrile en acide adipique [Bengis-Garber et al., Appl. Microbiol. Biotechnol., 1989, 32, II] et aussi *Rhodococcus rhodochrous* K22 dont la nitrilase permet l'hydrolyse de l'adiponitrile et du glutaronitrile [Yamada et al., J. Bacteriol., 1990, 172 (9), 4807-4815], cependant avec un rapport d'activité faible comparativement à celui de l'hydrolyse des nitriles aromatiques.

Par conséquent, on voit que l'hydrolyse enzymatique des dinitriles est assez complexe: dans tous les cas, la première fonction CN est bien hydrolysée par l'enzyme, alors que la deuxième fonction ne l'est pas du tout dans certains cas ou bien avec une vitesse très faible dans d'autres.

On a maintenant trouvé qu'il était possible d'hydrolyser totalement et rapidement les nitriles en carboxylates et plus particulièrement les dinitriles en carboxylates ou dicarboxylates en faisant appel à des enzymes convenablement sélectionnées, utilisées soit telles quelles, soit, de préférence, sous la forme de micro-organismes recombinés les générant.

La présente invention a donc pour objet de nouveaux polypeptides ayant une activité nitrilase, qui ont été isolés à partir d'une souche de *Comamonas testosteroni.* Plus précisément, ces polypeptides sont préparés par extraction et purification à partir de cultures de micro-organismes naturels ou recombinants, la purification étant réalisée par une succession d'étapes consistant à préparer un extrait enzymatique à partir de la culture cellulaire, à précipiter cet extrait avec du sulfate d'ammonium et à le purifier par différentes étapes de chromatographie et filtration sur gel. Ces étapes, qui font appel à des techniques bien connues de l'homme du métier, sont décrites en détail dans les exemples illustratifs ci-après.

Par "activité nitrilase", on désigne dans la présente description la conversion directe d'un nitrile en carboxylate d'ammonium, l'amide correspondant n'étant pas un substrat de l'enzyme.

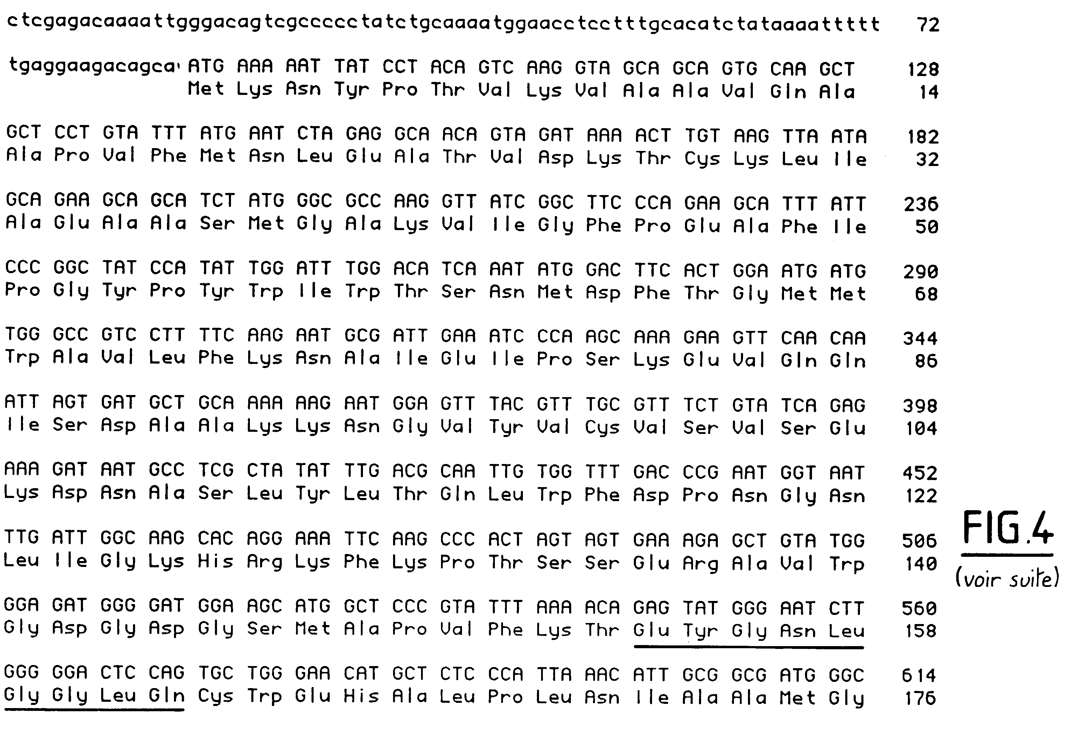

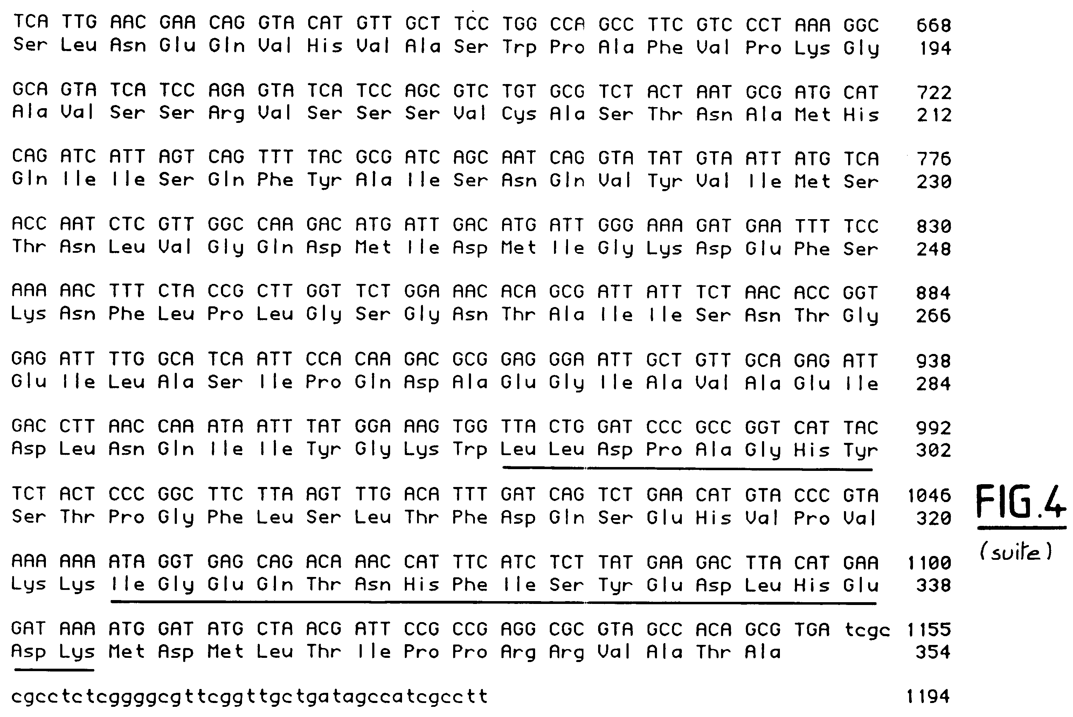

Un autre objet de l'invention concerne une séquence d'ADN codant pour un polypeptide ayant une activité nitrilase. La séquence d'ADN codant pour un polypeptide de l'invention peut être choisie parmi :
- la séquence d'ADN codant pour un polypeptide ayant une activité nitrilase, telle que représentée à la **fig. 4** et désignée par SEQ ID NO : 4 : dans la liste de séquences annexée,
- un analogue de cette séquence résultant de la dégénérescence du code génétique,
- ou bien une séquence d'ADN hybridant avec l'une de ces séquences ou avec un fragment de celles-ci et codant pour un polypeptide ayant une activité nitrilase.

Une telle séquence d'ADN peut être obtenue par clonage du fragment d'AdN génomique codant pour le polypeptide recherché, à l'aide de sondes nucléotidiques élaborées à partir du polypeptide purifié.

L'invention concerne également les cassettes d'expression qui portent, avec les signaux assurant son expression, la séquence d'ADN recombinant définie précédemment. Ces cassettes d'expression peuvent être soit intégrées dans le génome de l'hôte, soit localisées sur un vecteur d'expression, tel qu'un plasmide contenant un moyen de sélection.

Ces cassettes d'expression comportent notamment des régions d'initiation de la transcription et de la traduction, qui contiennent un site de fixation des ribosomes et une séquence promotrice. Ces régions peuvent être homologues ou hétérologues du micro-organisme produisant naturellement le polypeptide.

Le choix de ces régions dépend notamment de l'hôte utilisé. En particulier, lorsqu'il s'agit de micro-organismes hôtes procaryotes, le promoteur hétérologue peut être choisi parmi les promoteurs bactériens forts, tels que le promoteur de l'opéron tryptophane Ptrp de *E. coli*, le promoteur de l'opéron lactose Plac de *E. coli*, le promoteur droit du phage lambda P_{R}, le promoteur gauche du phage lambda P_{L}, les promoteurs forts de *Pseudomonas* et *Comamonas,* les promoteurs forts de *Corynébactéries.*

Plus particulièrement, dans le cas du promoteur droit du phage lambda, la forme thermosensible P_{R}CIts pourra être préférée. Dans le cas des micro-organismes eucaryotes, tels les levures, les promoteurs peuvent être issus de gènes glycolytiques de levure, tels les gènes codant pour la phosphoglycérate kinase (PGK), la glycéraldéhyde-3-phosphate déshydrogénase (GPD), la lactase (LAC4), l'énolase (ENO).

Concernant les sites de fixation des ribosomes, celui dérivé du gène CII de lambda ainsi que ceux dérivés de gènes homologues de *Comamonas* ou *Pseudomonas* ou ceux dérivés de gènes de *Corynebacteries* sont utilisés préférentiellement lorsque le micro-organisme hôte est procaryote.

Une région permettant une terminaison de la traduction et de la transcription fonctionnelle de l'hôte envisagé peut être positionnée en 3' de la séquence codante. La cassette d'expression comprend également un ou plusieurs marqueurs permettant de sélectionner l'hôte recombinant. Les marqueurs préférés sont des marqueurs dominants, c'est-à-dire conférant une résistance à des antibiotiques comme l'ampicilline ou la streptomycine ou à d'autres produits toxiques.

Parmi les micro-organismes hôtes utilisés, on peut citer notamment les entérobactéries telles que *E. coli,* les bactéries appartenant aux genres *Comamonas* ou *Pseudomonas*, les bactéries corynéformes telles que celles appartenant aux genres *Corynebactérium, Brevibacterium* ou *Rhodococcus.*

L'invention a également pour objet les micro-organismes contenant la séquence d'ADN recombinante selon l'invention, par exemple sur un plasmide comportant un moyen de sélection.

Un micro-organisme recombiné contenant ladite séquence d'ADN sur une structure plasmidique a été déposé à la Collection Nationale de Cultures de Micro-organismes (C.N.C.M.) (Institut Pasteur, 25 rue du Docteur Roux, Paris) sous le n°I-1242 le 21 juillet 1992. Ce micro-organisme est la souche *E. coli* TG1 qui contient le plasmide pXL2148 ; ce micro-organisme est également identifié par la Demanderesse par la référence G4207.

L'invention a également pour objet les micro-organismes susceptibles de transformer des nitriles en carboxylates et plus particulièrement des dinitriles aliphatiques de formule NC - R - CN dans laquelle R est un groupe alkylène linéaire ou ramifié ayant de 1 à 10 atomes de carbone en carboxylates.

Au-delà de l'acquisition de leurs structures I et II lors de leur synthèse par les micro-organismes selon l'invention, il est important que les polypeptides considérés se stabilisent dans leurs structures III et IV, de façon à être doués d'une activité nitrilase optimale.

Il est du mérite de la demanderesse d'avoir découvert des moyens pour favoriser la susdite stabilisation.

Ainsi, tout micro-organisme selon l'invention contient, de préférence :
- au moins un agent protéique d'aide au repliement des polypeptides qu'il synthétise et, en particulier, de la nitrilase dont il est question dans le présent exposé,
- et/ou les gènes codant pour un tel agent, cet agent étant présent dans une quantité supérieure à celle correspondant au niveau de base du micro-organisme considéré.

Par niveau de base, on entend, au sens de la présente invention, le niveau maximum pouvant être atteint par le micro-organisme sauvage correspondant considéré.

Avantageusement, cet agent est la chaperone GroE de *E. coli* ou son homologue d'origine eucaryote ou procaryote.

La chaperone GroE d'*E. coli* est normalement présente dans les souches sauvages.

Les gènes codant pour l'agent sont portés par le chromosome ou par un élément extra-chromosique (plasmide, phage). Il est préférable de les amplifier par tout moyen connu et approprié, de manière à favoriser la synthèse d'agent dans le micro-organisme.

Les gènes codant pour l'agent sont sous la dépendance de systèmes d'expression homologues ou hétérologues de leur micro-organisme hôte.

L'invention concerne aussi le procédé de transformation des nitriles en carboxylates à l'aide d'un polypeptide selon l'invention ou d'un micro-organisme recombiné le générant. Ce procédé consiste à mettre en présence le nitrile à transformer avec un polypeptide ou un micro-organisme recombiné tel que défini précédemment. On opère généralement à la température ambiante. Selon un mode particulier de réalisation de l'invention, le polypeptide ou le micro-organisme recombiné est immobilisé sur ou dans un support solide.

Le procédé de l'invention convient pour la transformation des nitriles en carboxylates et plus particulièrement pour la transformation en carboxylates des dinitriles de formule NC - R - CN dans laquelle R est un groupe alkylène linéaire ou ramifié contenant 1 à 10 atomes de carbone.

Le procédé de l'invention est particulièrement approprié pour la synthèse enzymatique de l'adipate d'ammonium à partir d'adiponitrile.

Les exemples qui suivent permettent d'illustrer les caractéristiques et les avantages de la présente invention, sans toutefois en limiter la portée.

### DESCRIPTION DES FIGURES

La **fig. 1** représente le rendement (%) d'hydrolyse de l'adiponitrile en cyanovalérate (courbe a) et en adipate d'ammonium (courbe b) en fonction du temps de réaction en heures pour la souche *Comamonas testosteroni* sp.

Les **fig. 2a** et **2b** représentent les cartes de restriction des plasmides pXL2075 et pXL2076.

La **fig. 3** représente la carte de restriction du fragment SstI-SstI de 4,1 kb contenant la séquence d'ADN (dénommée sur la figure "gène de la nitrilase") codant pour le polypeptide ayant l'activité nitrilase selon l'invention, fragment présent dans les plasmides pXL2075 et pXL2076. La stratégie d'élaboration du fragment XbaI-SstI contenant la séquence d'ADN selon l'invention est également représentée sur cette figure.

La **fig. 4** représente la séquence SEQ ID NO : 4 : d'ADN selon l'invention avec sa séquence d'acides aminés déduite.

La **fig. 5** représente la carte de restriction du plasmide pXL2087.

La **fig. 6** représente la carte de restriction du plasmide pXL2148.

La **fig. 7** représente l'électrophorèse sur gel de SDS-PAGE SDS à 10 % montrant l'expression de la séquence d'ADN selon l'invention dans la souche *E. coli* TG1/pXL2027. Chaque piste correspond à une quantité de protéine équivalente à 60 µl de culture à une densité optique à 610 nm de 3.

La **fig. 8** représente la carte de restriction du plasmide pXL2158.

La **fig. 9** représente l'électrophorèse sur gel de SDS-PAGE SDS à 12,5 % montrant l'expression de la séquence d'ADN selon l'invention dans les souches TG1/pXL2158 et TG1/pXL2158 + pXL2035 (GroE).

La **fig. 10** représente la carte de restriction du plasmide pXL 2169.

La **fig. 11** représente l'électrophorèse sur gel de SDS-PAGE SDS à 10 % montrant l'expression de la séquence d'ADN selon l'invention dans la souche *Pseudomonas putida* G2081 - pXL2169.

Les abréviations utilisées dans la suite de la description ont les significations ci-après :
- SSC :: tampon couramment utilisé pour des hybridations ; il contient du citrate de sodium et du NaCl. (20XSSC = NaCl 3M-citrate de sodium pH 7, 0,3 M)
- SDS :: dodécylsulfate de sodium
- FPLC :: chromatographie liquide dénommée en langue anglaise "fast protein liquid chromatography"
- SDS-PAGE :: électrophorèse en gel à base de dodécylsulfate de sodium et de polyacrylamide
- IPTG :: isopropyl β-D thiogalactopyranoside.

### EXEMPLES

### EXEMPLE 1 : PURIFICATION DE LA NITRILASE DE Comamonas testosteroni sp.

### 1 - PRÉPARATION DES CELLULES :

Une souche de *Comamonas testosteroni* sp a été cultivée, en fiole agitée, à 28°C, pendant 15 h 30, dans le milieu A dont la composition est la suivante : Milieu A
- Glucose 5 g/l
- (NH₄)₂SO₄ 1 g/l
- Na₂HPO₄ 5,24 g/l
- KHPO₄ 2,77 g/l
- Extrait de levure 5 g/l
- Casamino Acides 1 g/l

Cette préculture a servi à ensemencer un fermenteur de 20 l contenant 15 1 de milieu A. Le pH, la température, le débit d'air et la vitesse d'agitation ont été régulés respectivement à 6,6, 28 C, 300 l/h et 350 tr/min. Après 24 h, 84 g de cellules humides ont été récoltées. Cela correspond à une teneur en poids sec de cellules de 0,9 g/l et à une densité optique à 660 nm (DO_{660 nm}) de 2.

### 2 - DÉTERMINATION DE L'ACTIVITÉ ENZYMATIQUE SUR ADIPONITRILE :

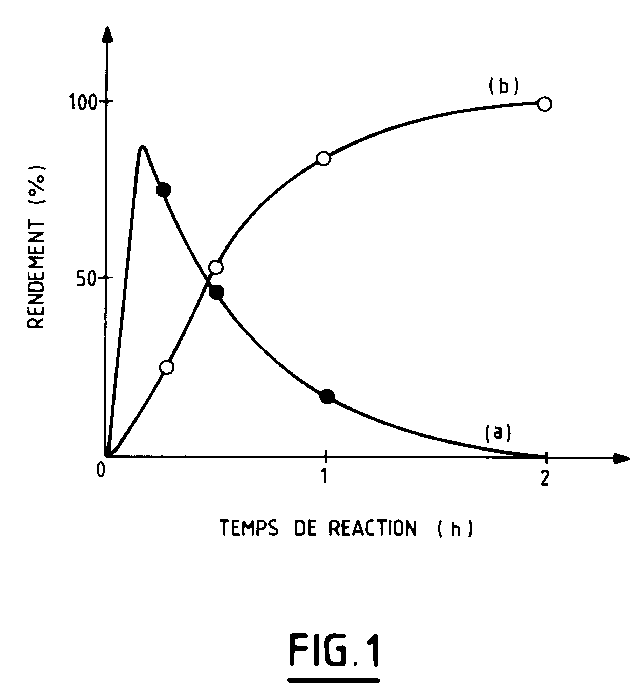

Un culot cellulaire contenant 13,1 mg de poids sec de cellules a été mis en suspension dans 2 ml d'une solution 52,3 mM en adiponitrile dans le tampon phosphate de potassium 50 mM ; pH 7. La réaction a été conduite à 25°C sous agitation et la cinétique a été suivie par prélèvement. Sur chaque prélèvement ont été dosés par chromatographie liquide haute performance (CLHP) le cyano-5-valéramide, l'adipamide, le cyano-5-valérate, l'adipamate et l'adipate. Les résultats sont regroupés sur la **fig. 1** qui représente les courbes de rendement (en ordonnées) en cyanovalérate (courbe a) et en adipate d'ammonium (courbe b). La vitesse de formation du cyanovalérate et de l'adipate était respectivement supérieure à 0,45 et égale à 0,15 U/mg de cellules poids sec (1 U est égale à 1 µmole de produit formé par minute).

### 3 - PURIFICATION :

Toutes les étapes de la purification ont été réalisées dans le tampon Tris/HCl 50 mM pH 7,5, 1 mM de dithioérythritol (DTE) sauf indication contraire. A chacune des étapes, l'activité nitrilase des fractions a été déterminée à pH 7 et à 25°C dans le tampon phosphate 10 mM en présence d'adiponitrile 10 mM. La concentration en protéine des pools a été déterminée par la méthode au bleu de Coomassie (kit PIERCE® Protein assay). Les protéines ont été analysées par électrophorèse sur gel de polyacrylamide-SDS (Phastsystem PHARMACIA®). Les modes opératoires de chacune des étapes sont commentés ci-dessous.
**Etape 1** : Extrait brut
   57 g de cellules humides ont été reprises dans 85 ml de tampon et traitées aux ultrasons pendant 30 min (Sonicateur VIBRACELL® de Bioblock : Sonde 13 mm ; puissance 7 ; 40 % du cycle actif). La DO_{660 nm} est passée ainsi de 97 à 60. Après centrifugation à 48 000 g maximum pendant 60 min, le surnageant a été récupéré.
   Ce surnageant a été amené, par adjonction progressive de sulfate d'ammonium, à 15 % de la saturation. Après 1 h, la suspension a été centrifugée 30 min à 30 000 g maximum. Le surnageant a été amené à 50 % de la saturation. Après 1 h, la suspension a été centrifugée dans les mêmes conditions, le précipité a été récupéré puis dialysé contre le tampon pendant deux jours.
**Etape 2** : Colonne échangeuse d'ions (Q Sepharose® Fast Flow)
   La fraction dialysée a été chargée à un débit de 125 ml/h sur une colonne (26 x 380 mm) de "Q Sepharose Fast Flow" équilibrée avec le tampon à un débit de 250 ml/h. La colonne a été percolée à un débit de 250 ml/h successivement par les solutions suivantes :
   - 166 ml de tampon
   - 180 ml d'un gradient 0 à 0,2 M en KCl dans le tampon
   - 180 ml de tampon additionné de KCl 0,2 M
   - 270 ml d'un gradient 0,2 à 0,4 M en KCl dans le tampon
   - 180 ml de tampon additionné de KCl 0,4 M
   - 200 ml de tampon additionné de KCl 1 M

   La fraction ayant l'activité nitrilase a été éluée dans un volume de 129 ml au cours du palier à 0,2 M en KCl.
   Les étapes suivantes sont réalisées sur le système FPLC (Pharmacia®).
**Etape 3** : filtration sur gel (FPLC Superdex® 200)
   La fraction ayant l'activité nitrilase obtenue précédemment (129 ml) a été concentrée jusqu'à 12 ml par précipitation des protéines avec du sulfate d'ammonium à 80 % de la saturation, suivie d'une dialyse contre le tampon. La fraction ainsi concentrée (12 ml) a été chargée en 2 fois sur la colonne de gel (16 x 600 mm) équilibrée avec le tampon additionné de 0,1 M de KCl à un débit de 0,8 ml/min. Les fractions ayant l'activité nitrilase ont été éluées avec le tampon ci-dessus à un débit de 1 ml/min et dans un volume total de 36 mi. Ces fractions correspondent à un poids moléculaire de 280 kDa.
**Etape 4** : Colonne d'hydroxyapatite (BIO-RAD HPHT® ; 7,8 x 100 mm)
   Les fractions obtenues ci-dessus ont été concentrées à 8 ml par ultrafiltration (membrane DIAFLO PM 39 AMICON®). La solution concentrée a été injectée sur la colonne d'hydroxyapatite équilibrée avec le tampon additionné de CaCl₂, 10 µM. La colonne a été percolée, à un débit de 0,5 ml/min, successivement avec :
   - 5 ml du tampon d'équilibrage,
   - 15 ml d'un gradient de phosphate de potassium de 0 à 350 mM dans le tampon d'équilibrage,
   - 10 ml du tampon d'équilibrage additionné de 350 mM de phosphate de potassium.

   Les fractions ayant l'activité nitrilase ont été éluées entre 62 et 135 mM en phosphate de potassium sur un volume de 3 ml.
**Etape 5** : Colonne d'interaction hydrophobe (FPLC-Phenyl Superose HR 5/5)
   Les fractions actives obtenues ci-dessus, amenées à 15 % de la saturation en sulfate d'ammonium, ont été chargées à un débit de 0,5 ml/min sur la colonne, équilibrée avec du tampon contenant du sulfate d'ammonium à 15 % de la saturation. La colonne a été percolée avec :
   - 6 ml de tampon d'équilibrage,
   - 12 ml d'un gradient décroissant de sulfate d'ammonium 15% à 0 % de la saturation en sulfate d'ammonium dans le tampon,
   - 23 ml de tampon.

   Une partie des fractions ayant l'activité nitrilase ont été éluées lors du lavage de la colonne avec le tampon d'équilibrage. Ces fractions actives ont été réinjectées dans les mêmes conditions. Cette opération a été effectuée deux fois. Les fractions actives, éluées après le gradient, ont été réunies (volume 51 ml).
**Etape 6** : Filtration sur gel (FPLC-Superdex® 200)
   Les 51 ml ont été concentrés à 3 ml par ultrafiltration sur membrane (AMICON DIAFLO® PM30). Ces 3 ml ont été chargés sur la colonne (16 x 600 mm), équilibrée avec le tampon additionné de 0,1 M KCl. Les 9 ml contenant l'activité ont été élués à une position correspondant à un poids moléculaire de 280 kDa. Cette solution a été amenée à 36 % de glycérol puis congelée pendant 15 jours.
**Etape 7** : Colonne échangeuse d'ions (FPLC Mono Q HR 5/5)
   La solution protéique a été décongelée et chargée sur la colonne, équilibrée avec le tampon contenant 0,1 M de KCl, à un débit de 0,5 ml/min. La colonne a été percolée successivement avec :
   - 15 ml à 0,5 ml/min de tampon additionné de 0,1 M en KCl,
   - 4,5 ml à 1 ml/min de tampon additionné de 0,1 M en KCl,
   - 15 ml à 1 ml/min d'un gradient de KCl de 0,1 à 0,4 M dans le tampon,
   - 10 ml de tampon additionné de 0,4 M de KCI.

   Les fractions actives ont été éluées entre 0,15 et 0,3 M de KCl. Ces fractions sont homogènes. Par analyse SDS-PAGE, deux bandes très proches de 38 et 39 kDa sont visibles. Les fractions ainsi obtenues seront dénommées ci-après "nitrilase purifiée".
   Les données de chacune des étapes de purification ci-dessus sont rassemblées dans le tableau 1 ci-après :

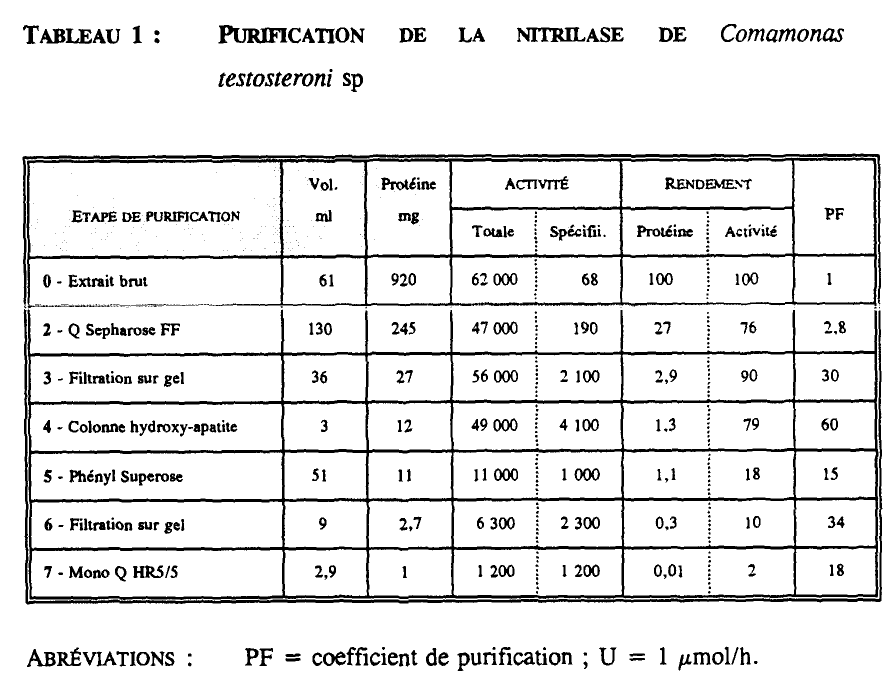

### 4 - DÉTERMINATION DE LA SÉQUENCE N-TERMINALE DE LA NITRILASE

A partir de la protéine purifiée, la séquence N-terminale de 27 aminoacides a été déterminée par dégradation séquentielle automatique d'Edman en utilisant un appareil "Applied Biosystems Model 470 A". Cette séquence désignée par SEQ ID NO : 1 : dans la liste de séquence annexée, est la suivante :

suivante :
Met Lys Asn Tyr Pro Thr Val Lys Val Ala Ala Val Gln Ala Ala V

Phe Met Asn Leu Glu Ala Thr Val Asp Lys Thr
20 . 25

La recherche dans les banques de séquences a permis de trouver une identité de 53 % avec la nitrilase de *Klebsiella pneumoniae* active sur le Bromoxynile, qui fait l'objet de la demande de brevet EP N° 373 173.

### 5 - ACTIVITÉ DE LA NITRILASE PURIFIÉE :

a) - Influence du pH sur l'activité de la nitrilase :
   La nitrilase purifiée a été testée à différents pH sur deux substrats adiponitrile et cyano-5-valérate dans les conditions indiquées dans le tableau 2 ci-après. 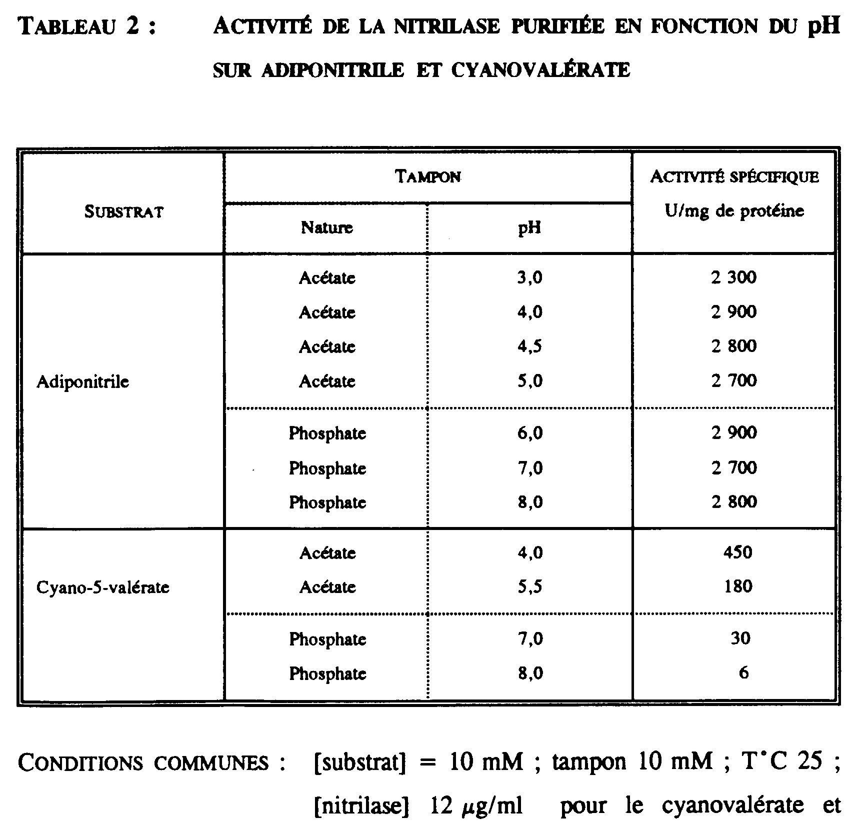 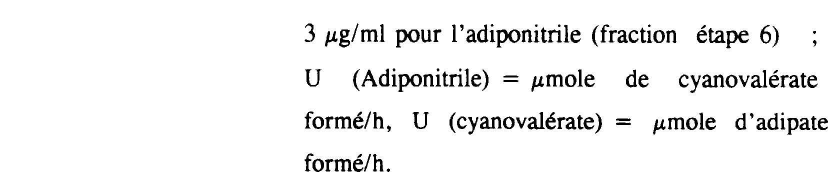
b) - Spectre d'activité de la nitrilase purifiée :
   Les activités de la nitrilase purifiée ont été mesurées sur adiponitrile, cyano-5-valéramide, acide cyano-5-valérique, benzonitrile, propionitrile, acrylonitrile. Les résultats sont donnés dans le tableau 3.

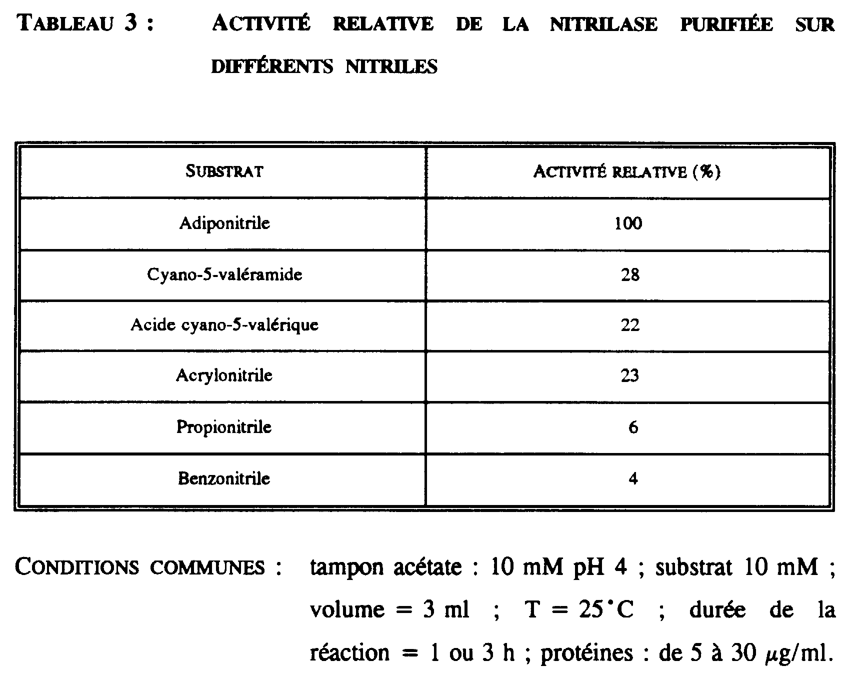

### EXEMPLE 2 : CLONAGE DE LA NITRILASE DE Comamonas testosteroni sp.

A partir de la séquence NH₂ terminale présentée dans l'exemple 1, une sonde nucléotidique a été synthétisée ; le pourcentage en GC élevé des souches de *Comamonas* décrites dans la littérature (Tamaoka et al., Int. J. Syst. Bacteriol, 1987, 37, 52-59) a dicté un choix pour la troisième position du codon dans le cas des lysines et dans le cas de la valine. La sonde est un 26 mer 128 fois dégénéré (dans la séquence de bases nucléotidiques N remplace A, C, G ou T) :

M K N Y P T V K V Acides Aminés
5' ATGAAGAATT ATCCNACNGT CAAGGT 3' Bases nucléotidiques
C C G Variants

La séquence d'acides aminés MKNYPTVKV donnée ci-dessus, correspond à la SEQ ID NO : 2 : dans la liste de séquences annexée.

La stratégie suivie a consisté tout d'abord à vérifier la spécificité de cette sonde nucléotidique et à déterminer la nature des fragments d'AdN génomique à cloner. Brièvement, l'AdN génomique de *Comamonas testosteroni sp.* a été digéré par plusieurs enzymes de restriction (SstI, SphI, BamHI, PstI...) correspondant à des sites utilisables pour le clonage.

Après électrophorèse sur gel d'agarose et transfert sur membrane de Nylon, les diverses digestions ont été hybridées à la sonde. On constate que la sonde a une spécificité suffisante dans les conditions d'hybridation utilisées (tampon d'hybridation = 5x SSC, 5x Denhardt, 0,1 % SDS, 50 mM Na₃PO₄ pH 6,5, 250 µg/ml ssAdN ; température d'hybridation 50°C. Conditions de lavage : 1 h, 6x SSC, température ambiante et 5 min en 2x SSC, 0,1 % SDS à 50°C).

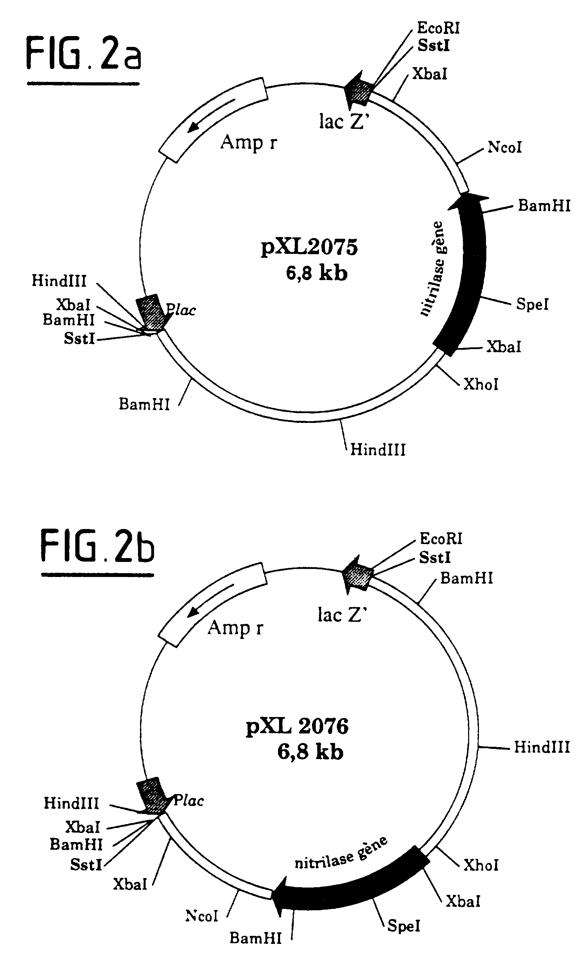

Dans ces conditions, la sonde a permis d'obtenir des signaux importants et sans ambiguïté ; en particulier, dans le cas des digestions par SstI, SphI, BamHI et PstI. Les empreintes d'hybridation montrent en particulier l'existence d'un fragment unique SstI-SstI d'environ 4 kb. Afin de cloner ce fragment, les fragments de 3,5 à 4,5 kb d'une digestion SstI de l'AdN génomique ont été purifiés par électrophorèse préparative sur agarose et électroélution, puis ligaturés au plasmide pUC19 (YANISCH et al., Gene 33 (1985)103), lui-même digéré par SstI. Après transformation dans la souche DH5α (Clontech Laboratory, Palo Alto Californie), 600 clones blancs sur LB amp X- gal (SAMBROOK et al., Molecular Cloning. A laboratory Manual, 2^{nd} édition, Cold Spring Harbor Laboratory Press, Cold Spring Harbor Laboratory N.Y. 1989) ont été repiqués individuellement, transférés sur membrane de Nylon, puis analysés par hybridation avec la sonde ayant servi pour hybrider le Southern blot et ce dans les mêmes conditions de stringence. Six clones ont ainsi été repérés comme hybridant très fortement avec la sonde. Deux clones ayant inséré le même fragment d'environ 4,1 kb dans les deux orientations (pXL2075 [**fig. 2a]** et pXL2076 [**fig. 2b])** ont été analysés plus en détail (cartographie de restriction, séquençage partiel en utilisant la sonde comme amorce et Southern blot). Il a ainsi pu être montré que la partie 5' du gène qui hybride avec la sonde est localisée sur un fragment de XhoI-XbaI de 150 pb environ orienté dans le sens XhoI vers XbaI. Les **fig. 2a** et **2b** représentent les cartes de restriction de ces plasmides.

### EXEMPLE 3 : SÉQUENCE D'UN FRAGMENT DE 1194 pb CONTENANT L'AdN CODANT POUR LE POLYPEPTIDE AYANT L'ACTIVITÉ NITRILASE.

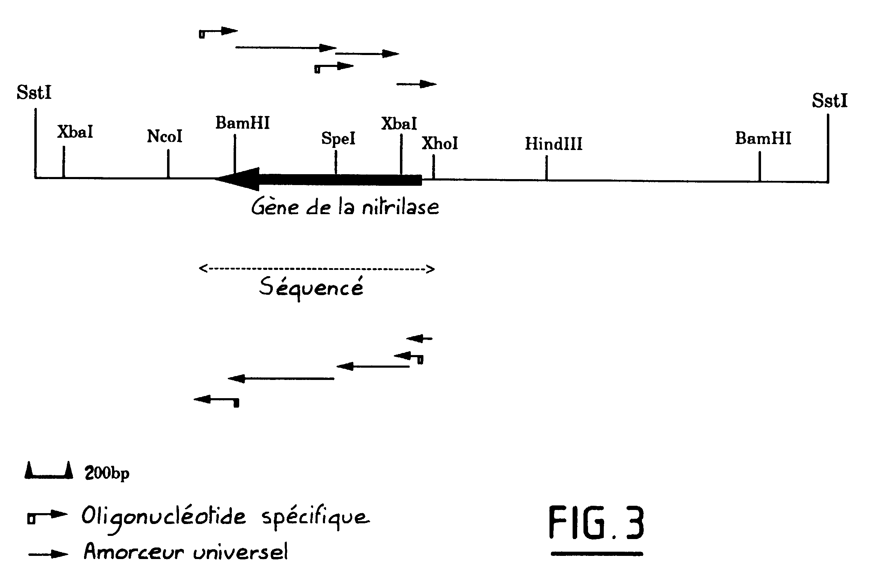

La localisation sur l'insert cloné du fragment de 1194 pb contenant le gène de la nitrilase séquencé est indiquée sur la **fig. 3.** La stratégie de séquençage de ce fragment réalisé selon des méthodes classiques connues de l'homme de métier est également indiquée sur la **fig. 3.** Les diverses séquences ont toutes été obtenues par la méthode de terminaison de chaînes (kit sequenase en présence de 7-deaza dGTP ; (³⁵S)dATP soit sur des matrices simple brin de M13 (mp 18 ou 19, voir YANISCH et al., op. cité) recombinant portant des sous-fragments, soit directement sur le plasmide pXL2075). Plusieurs amorces spécifiques ont également été synthétisées dans ce but.

La séquence d'ADN SEQ ID NO : 4 : selon l'invention est représentée sur la **fig. 4**. Le pourcentage en G+C moyen de la séquence obtenue est de 45,7 % ce qui est inférieur au pourcentage G+C de 61,5 % décrit chez d'autres souches de *Comamonas* (Tamaoka et al., op. cité). Une analyse de la séquence obtenue a permis de caractériser une phase ouverte de 1064 pb, appelée ci-après gène nit, codant pour un polypeptide de 354 résidus correspondant à un poids moléculaire de 38 725 Da. La séquence en acides aminés de ce polypeptide est indiquée sur la SEQ ID NO : 4 :, sur la SEQ ID NO : 5 : de la liste de séquences annexée et sur la **fig. 4.** Ce polypeptide comprend la séquence NH₂ terminale utilisée pour synthétiser la sonde ainsi que trois séquences internes déterminées sur des fragments trypsiques de la nitrilase purifiée (ces séquences internes sont soulignées sur la **fig. 4).**

Ainsi, cette phase ouverte représente la séquence d'ADN selon l'invention.

### EXEMPLE 4 : HOMOLOGIE AVEC D'AUTRES PROTÉINES, IDENTIFICATION DE SÉQUENCE HOMOLOGUE.

La séquence d'ADN selon l'invention a été comparée avec l'ensemble des séquences de la banque de protéines NBRF ; seule une homologie significative a été trouvée avec la Nitrilase de *Klebsiella ozaenae* spécifique de l'herbicide Bromoxynil (Stalker et al., J. Biol. Chem., 1988, 263, 6310-6314). Les deux nitrilases présentent une homologie stricte de 34,9 % répartie sur 320 acides aminés. Par ailleurs, cette protéine présente 34,4 % d'homologie stricte répartie sur 312 acides aminés avec la nitrilase *d'Arabidopsis* spécifique de l'indole-3-acétonitrile [Bartling & al., Eur. J. Biochem. 205, 417-424, 1992].

### EXEMPLE 5 : EXPRESSION DE LA NITRILASE DANS E. Coli.

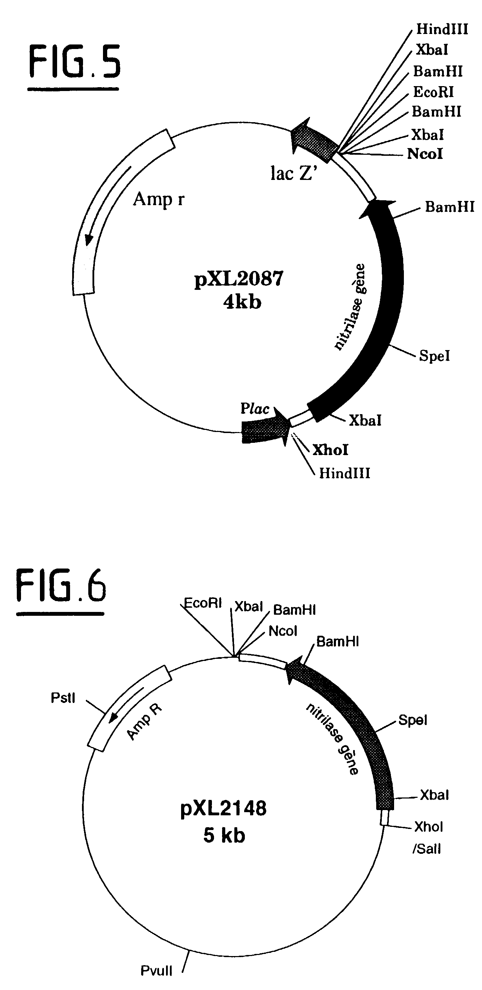

Afin de confirmer l'identification de la phase codante avec la nitrilase purifiée, le gène nit, précédé de son propre site de fixation des ribosomes, a été placé sous le contrôle du promoteur de l'opéron lactose de *E. coli* selon le mode opératoire décrit ci-après : le plasmide pXL2087, décrit sur la **fig. 5,** a été obtenu par insertion du fragment XhoI-NcoI dérivé du plasmide pXL2075 entre les sites correspondants du vecteur pMTL25 (Chambers et al., Gene, 1988, 68, 139-149). Ce plasmide contient donc le promoteur de l'opéron lactose Plac, suivi du site de fixation des ribosomes et du gène structural de la nitrilase, ainsi qu'un gène conférant la résistance à l'ampicilline.

L'expression de la nitrilase a été visualisée chez la souche E. coli TG1 contenant le plasmide pXL2087. Dans ce but, la souche TG1/pXL2087, ainsi que la souche témoin TG1/pUC19, ont été cultivées 16 h en milieu LB à 37°C (Miller, J.H. 1972, Experiments in Molecular Genetics - Cold Spring Harbor Laboratory, Cold Spring Harbor, N.Y.) contenant 100 µg/ml d'ampicilline, puis diluées au 100ème dans le même milieu et à la même température. Lorsque les cultures ont atteint une DO₆₁₀ comprise entre 0,5 et 1, de l'IPTG, à la concentration finale de 1 mM a été ajouté. Après 2 h de culture, les bactéries ont été collectées.

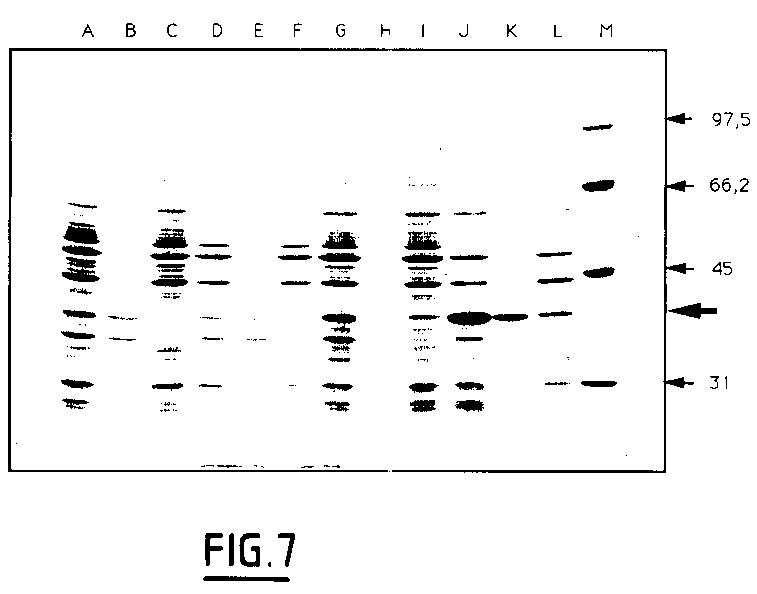

L'expression de la nitrilase a été mesurée, après sonication des cellules, en gel SDS-PAGE, dans la fraction brute ou après centrifugation dans le culot et dans le surnageant. Les résultats sont présentés sur la **fig. 7** et montrent un niveau élevé d'expression de la nitrilase dans les extraits de cellules cultivées en présence d'IPTG ; toutefois, cette protéine est essentiellement sous forme insoluble.

Sur la **fig. 7**, M représente le marqueur de poids moléculaires ; ceux-ci sont indiqués en kDa. D'autre part, les pistes ont les significations ci-après :

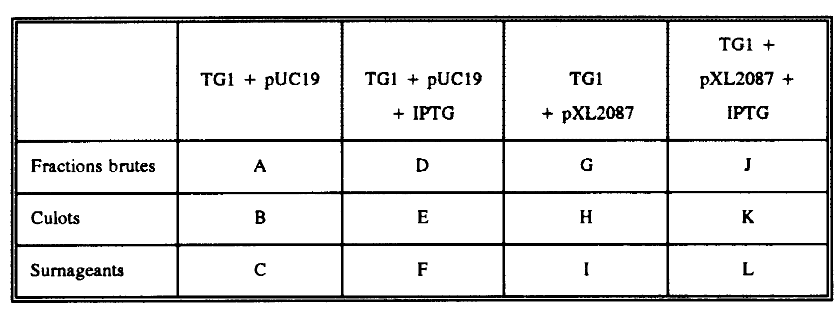

A partir du plasmide pXL2087, on a préparé le plasmide pXL2148 par insertion du fragment XhoI-EcoRI du plasmide pXL2087, portant le gène codant pour la nitrilase entre les sites SalI et EcoRI de pBR322 [SUTCLIFFE, Nucleic Acid Res. 5, (1978): 2721-2730].

Ce plasmide pXL2148, dont la carte de restriction est représentée sur la **fig. 6,** a été également utilisé pour transformer la souche de *E. coli* TG1 selon la méthode du chlorure de calcium. La sélection des micro-organismes a été faite sur ampicilline. La souche *E. coli* TG1 (pXL2048) (G4207) ainsi transformée a été déposée à la Collection Nationale de Cultures de Micro-organismes à Paris (Institut Pasteur, 25 rue du Docteur Roux) sous le n° 1-1242 le 21 juillet 1992.

D'autres systèmes d'expression ont été utilisés pour produire la nitrilase dans un micro-organisme recombinant.

Tout d'abord, le gène nit a été exprimé dans *E. coli* derrière le promoteur de l'opéron tryptophane de *E. coli*, sous la dépendance du RBS du gène CII du phage λ. Pour ce faire, un site de restriction NdeI a été créé sur le codon d'initiation de nit et le fragment NdeI/AhaII de 117 pb contenant la partie 5' du gène nit a _{é}té amplifié par la technique de PCR, à partir de pXL2087. Un fragment NdeI/XbaI de 61 pb, ₒbtenu après digestion du premier fragment, a été ligué au fragment EcoRI/NdeI contenant le promoteur de l'opéron tryptophane de *E. coli* et le site de fixation des ribosomes du gène CII du bactériophage λ (Ptrp-RBSCII) entre les sites EcoRI et XbaI de pUC19 (Yanisch et al., Gene 33 (1985) 103) pour aboutir au plasmide pXL2149. Le fragment EcoRI/XbaI de pXL2149, contenant la partie 5' de nit derrière Ptrp-RBSCII, a été ligué avec le fragment XbaI/SalI de pXL2087 contenant la partie 3' du gène nit entre les sites EcoRI et SalI de pXL642 (Mayaux, resultats non publiés) : pXL642 est un derivé de pXL534 (Latta et al., 1990. DNA Cell Biol., 9, 129) dans lequel le gène surexprimé code pour un inhibiteur tissulaire de métallo-proteases et dans lequel le site HindIII en aval du gène surexprimé a été remplacé par le multisite EcoRI/HindIII de M13mp18.

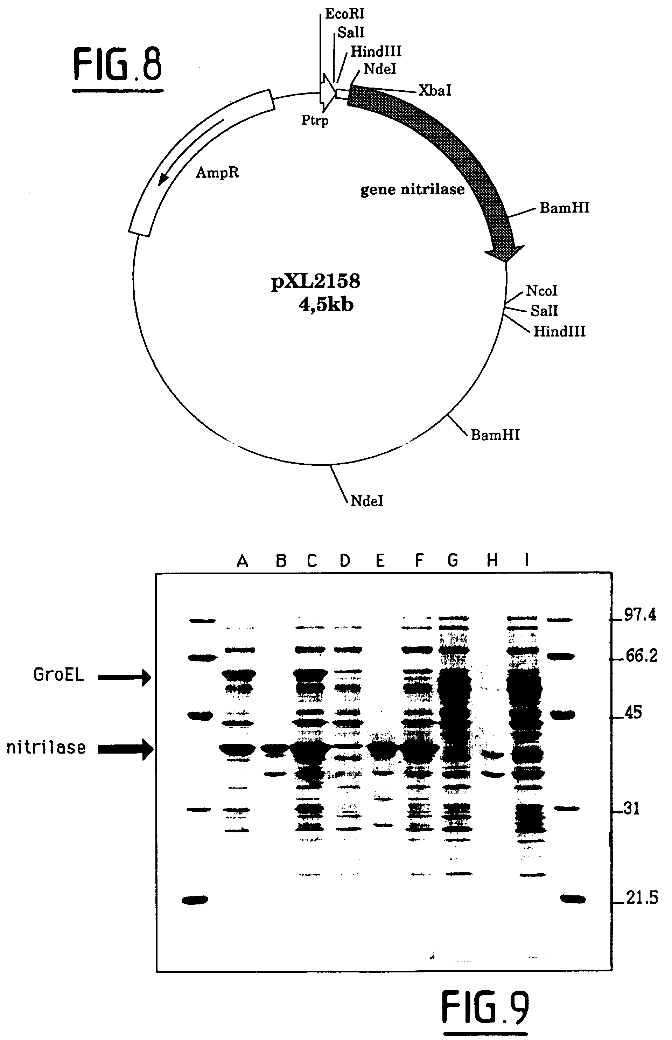

Le plasmide pXL2158 final est donc un dérivé de pBR322 (Sutcliffe, Nucleic Acid Res. 5, (1978) : 2721) contenant un gène conférant la résistance à l'ampicilline et le gène nit sous controle de Ptrp-RBSCII. La carte de restriction de ce plasmide pXL2158 est représentée à la **fig. 8.**

Le plasmide pXL2158 a été utilisé pour transformer la souche *E. coli* TG1. La souche TG1/pXL2158, ainsi que la souche témoin TG1 contenant le vecteur pMTL22, ont été cultivées 16 h en milieu M9 glucose à 30°C (Miller, J.H. 1972. Experiments in Molecular Genetics. Cold Spring Harbor Laboratory, Cold Spring Harbor, N.Y.) contenant 100 µg/ml d'ampicilline et 100 µg/ml de tryptophane. Ces cultures ont été diluées 100 fois dans le même milieu en absence de tryptophane et cultivées pendant 6 heures à la même température.

L'expression de la nitrilase de *Comamonas testosteroni* NI 1 a été mesurée, après sonication des cellules, en gel SDS-PAGE 12,5%, dans la fraction brute ou, après centrifugation, dans le culot et dans le surnageant. Les resultats sont présentés sur la **fig. 9.**

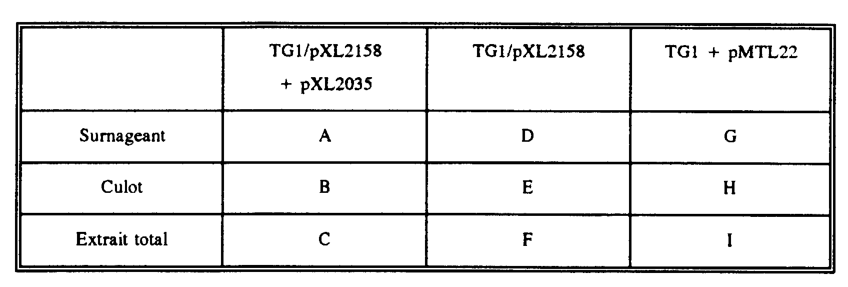

On voit sur ce gel que pXL2158 induit une forte expression de la nitrilase majoritairement sous forme insoluble.

L'efficacité de la chaperone GroE a été alors testée (Hemmingsen et al., 1988. Nature. 333 : 330) pour aider au repliement correct de la nitrilase. Pour ce faire, le plasmide pXL2035 a été construit de la manière suivante. Le fragment EcoRI/HindIII de 2,2 kb contenant les gènes groES et groEL, codant pour les deux sous-unités de GroE, a été extrait du plasmide pOF39 (Fayet et al., 1986. Mol. Gen. Genet. 202: 435) et introduit entre les sites EcoRI et HindIII du vecteur pDSK519 (Keen et al., 1988. Gene. 70 : 191).

Le plasmide pXL2035 a été introduit dans la souche TG1 contenant pXL2158. La souche résultante a été cultivée dans les mêmes conditions que précédemment, en présence de kanamycine 50 mg/l ; les résultats d'expression sont visualisés sur la **fig. 9.** Il ressort que la surexpression de GroE (seule la sous-unité GroEL est visible sur le gel) solubilise une majorité de la nitrilase exprimée à partir de pXL2158.

Le même système d'expression a été utilisé pour produire la nitrilase chez *Pseudomonas putida.* Ainsi, à partir de pXL2158, les fragments NdeI/NcoI de 1256 pb et NcoI/BamHI de 535 pb ont été introduits entre les sites NdeI et BamHI de pXL1841. pXL1841 (Blanche et al., 1991. J. Bacteriol. 173:4637) est un dérivé de pKT230 (Bagdasarian et al., 1981. Gene. 15: 237) exprimant un gène de *Methanobacterium ivanovii* derrière Ptrp-RBSCII.

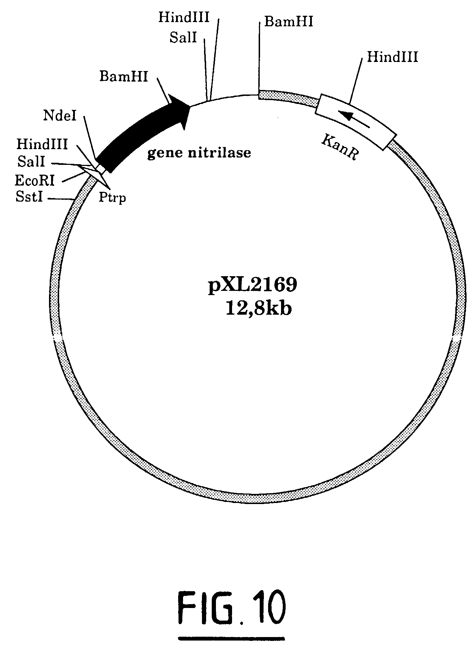

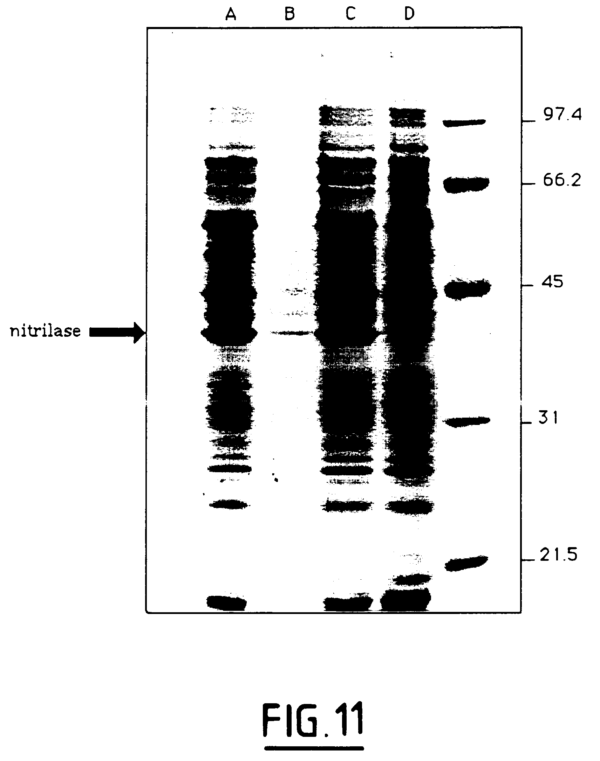

Le plasmide pXL2169 final est donc un dérivé de pKT230 contenant un gène conférant la résistance à la kanamycine et le gène nit sous contrôle de Ptrp-RBSCII (cf **fig. 10**). Ce plasmide a été introduit dans la souche de *Pseudomonas putida* G2081. G2081 est une dérivée de *Pseudomonas putida* KT2440 (Bagdasarian and Timmis, 1981. In Hofschneid and Goebel. Topics in Microbiology and Immunology. 47. Springer Verlag. Berlin.) rendue résistante à l'acide nalidixique et à la rifampicine. Le vecteur pDSK519 (Keen et al., 1988, Gene. 70 : 191) a été utilisé comme plasmide témoin. G2081 (pXL2169) et la souche G2081 (pDSK519) ont été cultivées la nuit à 30°C en milieu LB contenant 20 mg/l de kanamycine. Ces précultures ont été diluées 100 fois dans le même milieu. Les cultures ont alors été poursuivies 7 h 30 min à la même température. L'expression de la nitrilase de *Comamonas testosteroni* NI 1 a été mesurée, après sonication des cellules, en gel SDS-PAGE 10 %, dans la fraction brute ou, après centrifugation, dans le culot et dans le surnageant. Les résultats sont présentés sur la **fig. 11**. Seul l'extrait brut de la souche G2081 (pDSK519) a été déposé (puits D). Pour la souche G2081 (pXL2169) l'extrait total, le culot de sonication et le surnageant de sonication ont été déposés, respectivement, dans les puits C, B et A. Il ressort de cette expérience que la souche de *Pseudomonas putida* exprime des quantités importantes de nitrilase sous forme soluble.

### EXEMPLE 6: DOSAGE DE L'ACTIVITÉ NITRILASE DE SOUCHES RECOMBINANTES.

Les exemples qui suivent illustrent l'activité nitrilase des souches recombinantes de *E. coli* TG1 et de *Pseudomonas putida* G2081.

Les différents plasmides intégrés dans ces souches sont les suivants.

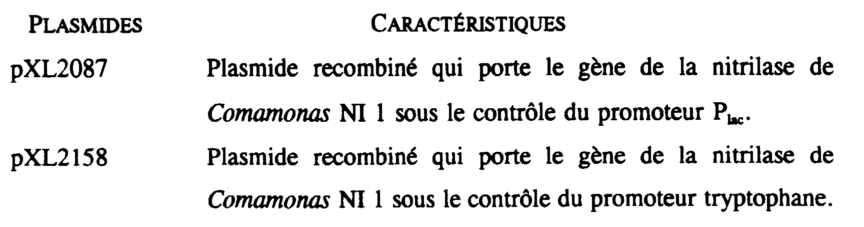

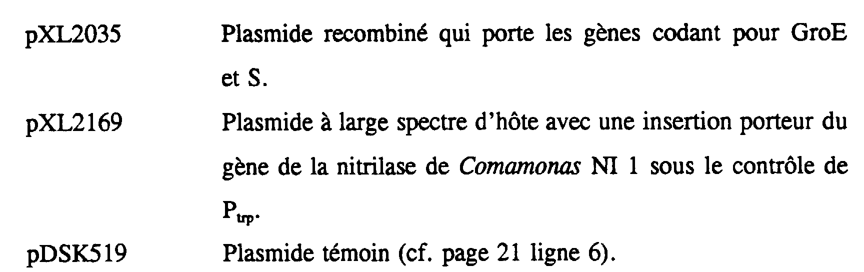

Les activités de ces souches, induites ou non, sont mesurées sur adiponitrile et cyano-5-valérate, à différents pH et comparées aux souches témoins négatif : *E. coli* TG1, *E. coli* TG1 (pXL2035) et *Pseudomonas putida* G2081.

### 1 - PRÉPARATION DES CELLULES :

Les cultures sont réalisées dans les conditions décrites dans le tableau 4. Au cours de la phase exponentielle de croissance, une des deux cultures de la souche recombinante est induite par l'IPTG 1 mM ; après 2 h à 37°C, cette culture est traitée.

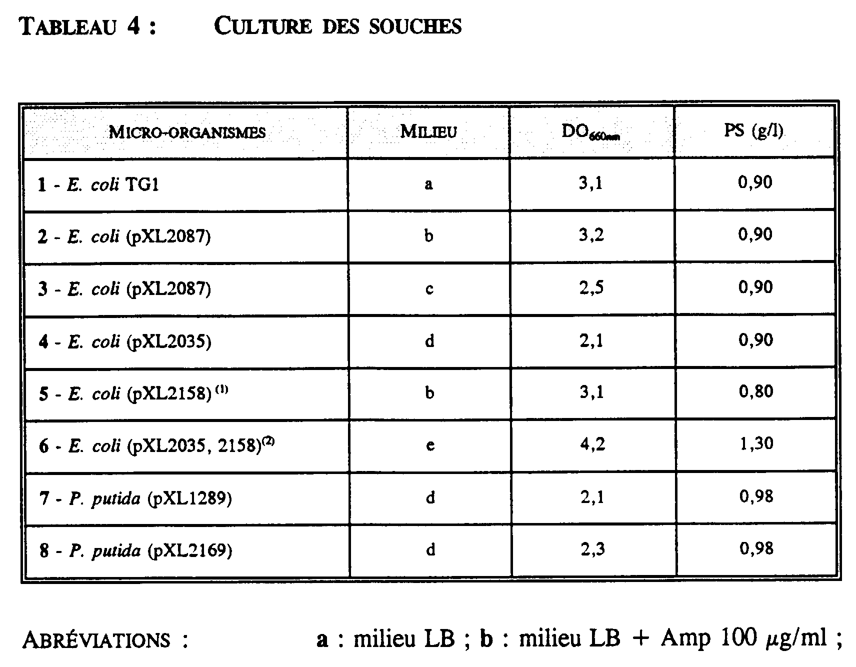

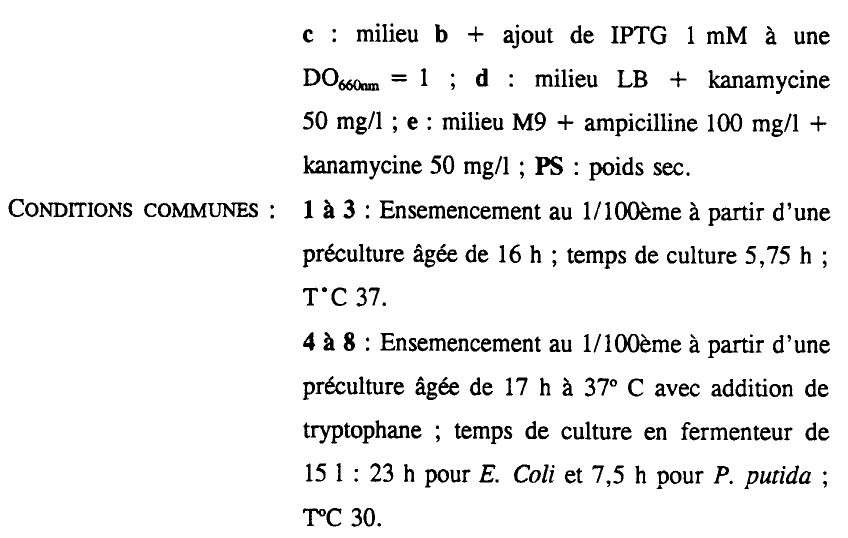

### 2 - MESURES DES ACTIVITÉS SPÉCIFIQUES :

Les conditions de mesures des activités spécifiques et les résultats sont répertoriés dans le tableau 5.

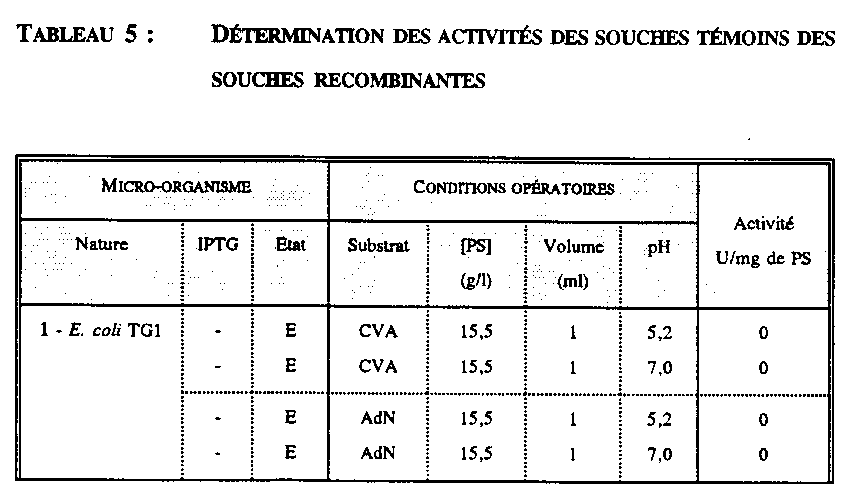

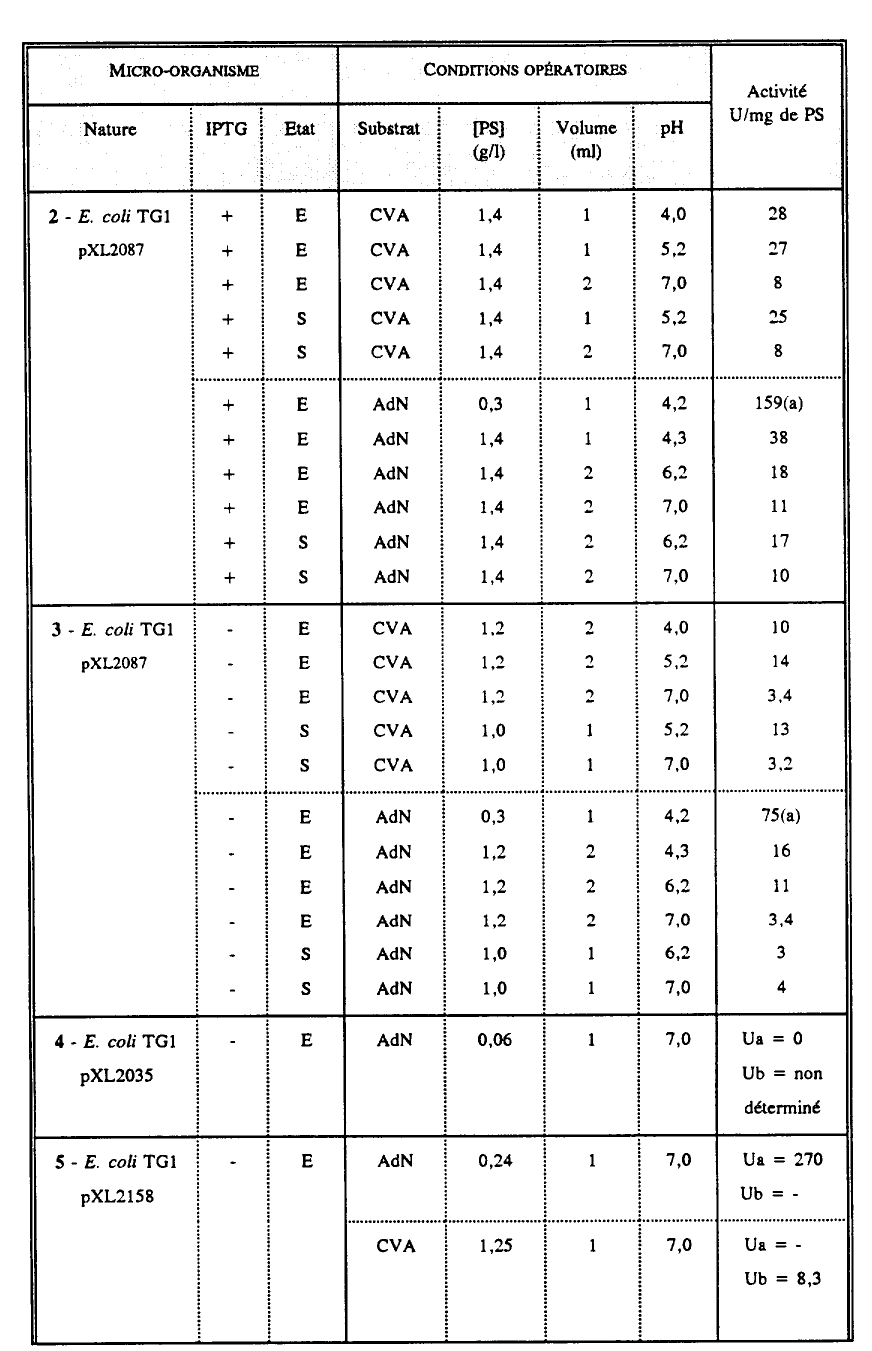

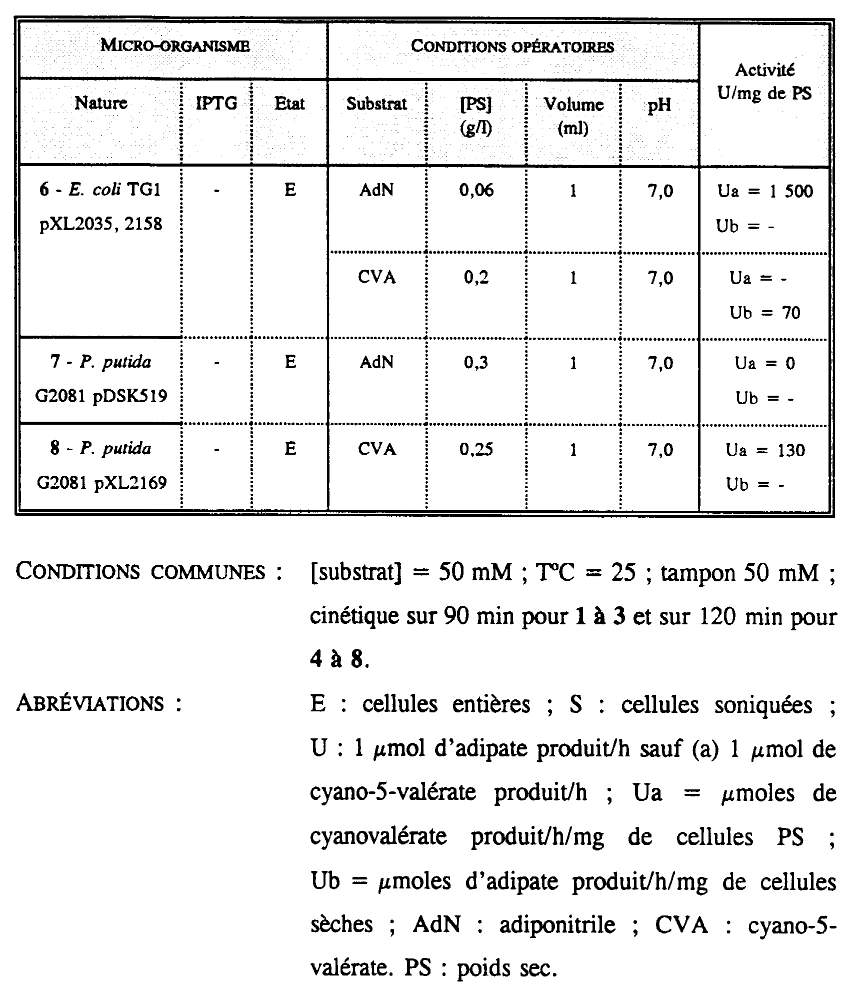

### EXEMPLE 7: SYNTHÈSE D'ADIPATE D'AMMONIUM PAR HYDROLYSE D'ADIPONITRILE EN BATCH PAR E. coli (pXL2087) EN SUSPENSION.

Dans un volume initial de 5 ml de tampon phosphate 50 mM pH 7 contenant la souche *E. coli* (pXL2087) à une concentration initiale de 21 g/l, on a ajouté à 25°C et sous agitation magnétique 120 µl ou 1 068 µmoles d'adiponitrile à 0, 1, 2, 3, 5, 6 et 7 h de réaction. Le suivi analytique a été assuré par prélèvements de 100 µl du volume réactionnel toutes les heures. On a constaté que l'hydrolyse se fait sans perte notable de la cinétique.

Les activités moyennes calculées sur 30 min après ajout de l'adiponitrile sont rassemblées dans le tableau 6 ci-après.

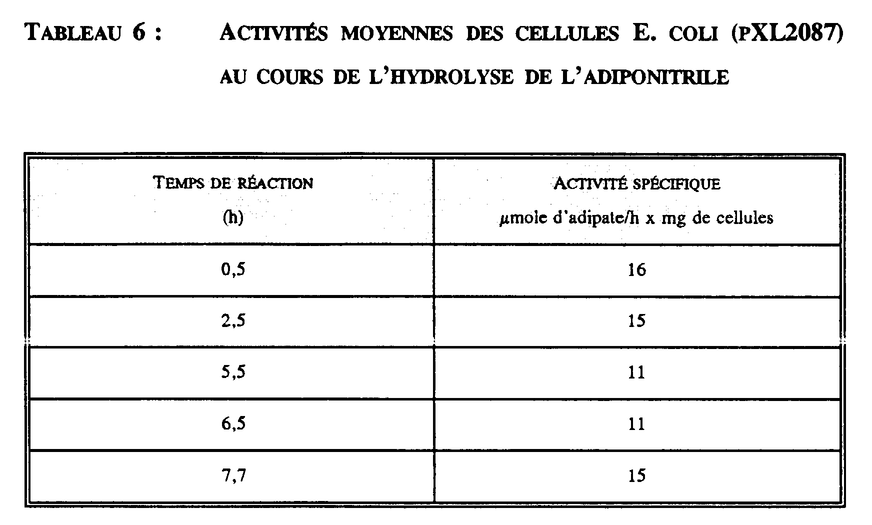

### EXEMPLE 8 : SYNTHÈSE D'ADIPATE D'AMMONIUM PAR HYDROLYSE DE L'ADIPONITRILE EN RÉACTEUR À LIT FIXE PAR E. Coli (pXL2087) IMMOBILISÉE SUR RÉSINE.

Les cellules d'*E. coli* (pXL2087) ont tout d'abord été fixées selon la technique décrite dans le brevet U.S. 4 732 851.

Le biocatalyseur résultant a ensuite été utilisé en colonne à lit fixe pour l'hydrolyse de l'adiponitrile en adipate d'ammonium.

### 1 - FIXATION DE E. coli (pXL2087) SUR RÉSINE.

Les cellules ont été fixées selon le protocole suivant :
- 1 g (poids humide) de *E. coli* (pXL2087) à 22 % de matières sèches,
- 1 g de polyazétidine POLYCUP,
- 1 g de résine DUOLITE A 171.

Le gramme de cellules a été mis en suspension dans la solution de polyazétidine. Après homogénéisation, la résine a été coulée dans la suspension cellulaire. L'ensemble a été agité à la spatule puis abandonné pendant 18 h, à l'air libre, sous une hotte, pour séchage. On a recueilli ainsi 4 ml ou 1,3 g de biocatalyseur.

Les activités des cellules immobilisées et libres ont été déterminées, à 25°C pH 7 sur une solution d'adiponitrile 50 mM. Elles sont respectivement de 30 et 110 µmoles de cyano-2-valérate/h/mg de cellules PS. On en déduit un rendement de fixation de 26 %.

### 2 - Hydrolyse de l'adiponitrile en réacteur à lit fixe :

La détermination du temps de demi-vie est réalisée en réacteur à lit fixe, continûment alimenté dans les conditions indiquées ci-après :

T°C 28 ; Catalyseur 0,5 g ou 2 ml ou 85 mg de cellules (poids sec) ; [adiponitrile] 50 mM ; tampon phosphate 50 mM pH 7 ; débit 3,7 +/- 0,1 ml/h ; colonne : diamètre 1 cm, hauteur 3 cm.

L'activité initiale des cellules était de 1,5 µmoles d'adipate/h/mg de cellules (poids sec). 66 % de l'activité initiale sont conservés après 32 jours ou 770 h.

## Revendications

1. Séquence d'ADN codant pour un polypeptide ayant une activité nitrilase, capable d'hydrolyser les nitriles en carboxylates,
caractérisée en ce qu'elle est choisie parmi :
- la séquence d'ADN codant pour un polypeptide ayant une activité nitrilase telle que représentée sur la **fig. 4** et désignée par SEQ ID NO : 4 : dans la liste de séquences annexée,
- un analogue de cette séquence résultant de la dégénérescence du code génétique,
- une séquence d'ADN hybridant avec l'une de ces séquences ou un fragment de celles-ci et codant pour un polypeptide ayant une activité nitrilase.

2. Séquence d'ADN recombinant selon la revendication 1, caractérisée en ce qu'elle contient la séquence nucléotidique suivante (SEQ ID NO :4 :):

CTCGAGACAA AATTGGGACA GTCGCCCCCT ATCTGCAAAA TGGAACCTCC TTTGCACATC 60
TATAAAATTT TTTGAGGAAG ACAGCAATGA AAAATTATCC TACAGTCAAG GTAGCAGCAG 120
TGCAAGCTGC TCCTGTATTT ATGAATCTAG AGGCAACAGT AGATAAAACT TGTAAGTTAA 180
TAGCAGAAGC AGCATCTATG GGCGCCAAGG TTATCGGCTT CCCAGAAGCA TTTATTCCCG 240
GCTATCCATA TTGGATTTGG ACATCAAATA TGGACTTCAC TGGAATGATG TGGGCCGTCC 300
TTTTCAAGAA TGCGATTGAA ATCCCAAGCA AAGAAGTTCA ACAAATTAGT GATGCTGCAA 360
AAAAGAATGG AGTTTACGTT TGCGTTTCTG TATCAGAGAA AGATAATGCC TCGCTATATT 420
TGACGCAATT GTGGTTTGAC CCGAATGGTA ATTTGATTGG CAAGCACAGG AAATTCAAGC 480
CCACTAGTAG TGAAAGAGCT GTATGGGGAG ATGGGGATGG AAGCATGGCT CCCGTATTTA 540
AAACAGAGTA TGGGAATCTT GGGGGACTCC AGTGCTGGGA ACATGCTCTC CCATTAAACA 600
TTGCGGCGAT GGGCTCATTG AACGAACAGG TACATGTTGC TTCCTGGCCA GCCTTCGTCC 660
CTAAAGGCGC AGTATCATCC AGAGTATCAT CCAGCGTCTG TGCGTCTACT AATGCGATGC 720
ATCAGATCAT TAGTCAGTTT TACGCGATCA GCAATCAGGT ATATGTAATT ATGTCAACCA 780
ATCTCGTTGG CCAAGACATG ATTGACATGA TTGGGAAAGA TGAATTTTCC AAAAACTTTC 840
TACCGCTTGG TTCTGGAAAC ACAGCGATTA TTTCTAACAC CGGTGAGATT TTGGCATCAA 900
TTCCACAAGA CGCGGAGGGA ATTGCTGTTG CAGAGATTGA CCTTAACCAA ATAATTTATG 960
GAAAGTGGTT ACTGGATCCC GCCGGTCATT ACTCTACTCC CGGCTTCTTA AGTTTGACAT 1020
TTGATCAGTC TGAACATGTA CCCGTAAAAA AAATAGGTGA GCAGACAAAC CATTTCATCT 1080
CTTATGAAGA CTTACATGAA GATAAAATGG ATATGCTAAC GATTCCGCCG AGGCGCGTAG 1140
CCACAGCGTG ATCGCCGCCT CTCGGGGCGT TCGGTTGCTG ATAGCCATCG CCTT 1194

3. Polypeptides résultant de l'expression d'une séquence d'ADN selon l'une des revendications 1 ou 2, et possédant une activité nitrilase.

4. Polypeptide selon la revendication 3, caractérisé en ce qu'il comprend la séquence désignée par SEQ ID NO : 5 : dans la liste de séquences annexée et donnée ci-après :

Met Lys Asn Tyr Pro Thr Val Lys Val Ala Ala Val Gln Ala Ala Pro
1               5                   10                  15
Val Phe Met Asn Leu Glu Ala Thr Val Asp Lys Thr Cys Lys Leu Ile
            20                  25                  30
Ala Glu Ala Ala Ser Met Gly Ala Lys Val Ile Gly Phe Pro Glu Ala
        35                  40                  45
Phe Ile Pro Gly Tyr Pro Tyr Trp Ile Trp Thr Ser Asn Met Asp Phe
    50                  55                  60
Thr Gly Met Met Trp Ala Val Leu Phe Lys Asn Ala Ile Glu Ile Pro
65                  70                  75                  80
Ser Lys Glu Val Gln Gln Ile Ser Asp Ala Ala Lys Lys Asn Gly Val
                85                  90                  95
Tyr Val Cys Val Ser Val Ser Glu Lys Asp Asn Ala Ser Leu Tyr Leu
            100                 105                 110
Thr Gln Leu Trp Phe Asp Pro Asn Gly Asn Leu Ile Gly Lys His Arg
        115                 120                 125
Lys Phe Lys Pro Thr Ser Ser Glu Arg Ala Val Trp Gly Asp Gly Asp
    130                 135                 140
Gly Ser Met Ala Pro Val Phe Lys Thr Glu Tyr Gly Asn Leu Gly Gly
145                 150                 155                 160
Leu Gln Cys Trp Glu His Ala Leu Pro Leu Asn Ile Ala Ala Met Gly
                165                 170                 175
Ser Leu Asn Glu Gln Val His Val Ala Ser Trp Pro Ala Phe Val Pro
            180                 185                 190
Lys Gly Ala Val Ser Ser Arg Val Ser Ser Ser Val Cys Ala Ser Thr
        195                 200                 205
Asn Ala Met His Gln Ile Ile Ser Gln Phe Tyr Ala Ile Ser Asn Gln
    210                 215                 220
Val Tyr Val Ile Met Ser Thr Asn Leu Val Gly Gln Asp Met Ile Asp
225                 230                 235                 240
Met Ile Gly Lys Asp Glu Phe Ser Lys Asn Phe Leu Pro Leu Gly Ser
                245                 250                 255

Gly Asn Thr Ala Ile Ile Ser Asn Thr Gly Glu Ile Leu Ala Ser Ile
            260                 265                 270
Pro Gln Asp Ala Glu Gly Ile Ala Val Ala Glu Ile Asp Leu Asn Gln
        275                 280                 285
Ile Ile Tyr Gly Lys Trp Leu Leu Asp Pro Ala Gly His Tyr Ser Thr
    290                 295                 300
Pro Gly Phe Leu Ser Leu Thr Phe Asp Gln Ser Glu His Val Pro Val
305                 310                 315                 320
Lys Lys Ile Gly Glu Gln Thr Asn His Phe Ile Ser Tyr Glu Asp Leu
                325                 330                 335
His Glu Asp Lys Met Asp Met Leu Thr Ile Pro Pro Arg Arg Val Ala
            340                 345                 350
Thr Ala

5. Micro-organisme contenant la séquence d'ADN (SEQ ID NO : 4 :) selon les revendications 1 ou 2.

6. Micro-organisme contenant la séquence d'ADN (SEQ ID NO : 4 :) selon les revendications 1 ou 2 sur un plasmide comportant un moyen de sélection.

7. Micro-organisme constitué par la souche *E. coli* TG1 contenant le plasmide pXL2148, souche référencée G4207 et déposée dans la Collection Nationale de Cultures de Micro-organismes sous le n° I-1242.

8. Micro-organisme contenant une cassette d'expression constituée de la séquence d'ADN (SEQ ID NO : 4 :) selon la revendication 1 ou 2 sous la dépendance de signaux assurant l'expression de cette séquence dans le micro-organisme hôte.

9. Micro-organisme selon la revendication 8, caractérisé en ce qu'il comprend en amont de la séquence d'ADN (SEQ ID NO : 4 :) un site de fixation des ribosomes et une séquence promotrice homologue ou hétérologue du polypeptide produit.

10. Micro-organisme selon la revendication 9, caractérisé en ce que le promoteur est choisi parmi les promoteurs suivants : le promoteur de l'opéron tryptophane Ptrp de *E. coli,* le promoteur de l'opéron lactose Plac de *E. coli ;* le promoteur droit du phage lambda PR, le promoteur gauche du phage lambda PL, et les promoteurs forts de *Corynebacterium,* de *Comamonas* ou de *Pseudomonas.*

11. Micro-organisme selon la revendication 9, caractérisé en ce que le site de fixation des ribosomes est celui dérivé du gène CII du phage lambda ou ceux dérivés de gènes de *E. coli, Comamonas, Pseudomonas* ou *Corynebacterium.*

12. Micro-organisme selon les revendications 8 à 11, caractérisé en ce que la cassette d'expression est portée par un plasmide comportant un moyen de sélection.

13. Micro-organisme selon la revendication 12, caractérisé en ce que le moyen de sélection est un marqueur conférant la résistance à un antibiotique.

14. Micro-organisme selon les revendications 5 à 13, caractérisé en ce qu'il est choisi parmi les souches *E. coli, Comamonas, Corynebacterium, Brevibacterium, Rhodococcus, Pseudomonas.*

15. Micro-organisme selon l'une quelconque des revendications 5 à 14, caractérisé :
- en ce qu'il contient au moins un agent protéique d'aide au repliement des polypeptides qu'il synthétise et, de préférence, des polypeptides selon la revendication 3 ou la revendication 4 et/ou les gènes codant pour un tel agent,
- et en ce que cet agent est présent dans une quantité supérieure à celle correspondant au niveau de base du micro-organisme considéré.

16. Micro-organisme selon la revendication 15, caractérisé en ce que l'agent est la chaperone GroE de *E. coli* ou son homologue d'origine eucaryote ou procaryote.

17. Micro-organisme selon la revendication 15 ou la revendication 16, caractérisé en ce que les gènes codant pour l'agent sont portés par le chromosome ou par un élément extra-chromosique (plasmide, phage) et en ce qu'ils sont amplifiés.

18. Micro-organismes selon la revendication 17, caractérisés en ce que les gènes codant pour l'agent sont sous la dépendance de systèmes d'expression homologues ou hétérologues audit micro-organisme.

19. Procédé de transformation enzymatique de nitriles caractérisé en ce qu'il consiste à mettre en contact les nitriles avec un polypeptide ayant une activité nitrilase selon l'une quelconque des revendications 3 ou 4 ou un micro-organisme hôte selon l'une quelconque des revendications 5 à 18.

20. Procédé selon la revendication 19, caractérisé en ce que le nitrile est un dinitrile de formule NC - R - CN dans laquelle R est un groupe alkylène ayant de 1 à 10 atomes de carbone.

21. Procédé selon l'une des revendications 19 et 20, caractérisé en ce que le nitrile est l'adiponitrile.

## Claims

1. DNA sequence coding for a polypeptide having nitrilase activity, capable of hydrolyzing nitriles to carboxylates,
characterized in that it is chosen from:
- the DNA sequence coding for a polypeptide having nitrilase activity as depicted in Fig. 4 and designated SEQ ID NO : 4 : in the attached sequence listing,
- an analogue of this sequence resulting from the degeneracy of the genetic code,
- a DNA sequence that hybridizes with one of these sequences or a fragment of the latter and codes for a polypeptide having nitrilase activity.

2. Recombinant DNA sequence according to claim 1, characterized in that it contains the following nucleotide sequence (SEQ ID NO : 4 :):

CTCGAGACAA AATTCGGACA GTCGCCCCCT ATCTGCAAAA TGGAACCTCC TTTGCACATC 60
TATAAAATTT TTTGAGGAAG ACAGCAATGA AAAATTATCC TACAGTCAAG GTAGCAGCAG 120
TGCAAGCTGC TCCTGTATTT ATGAATCTAG AGGCAACAGT AGATAAAACT TGTAAGTTAA 180
TAGCAGAAGC AGCATCTATG GCGCCCAAGG TTATCGGCTT CCCAGAAGCA TTTATTCCCG 240
GCTATCCATA TTGGATTTGG ACATCAAATA TGGACTTCAC TGGAATGATG TGGCCCGTCC 300
TTTTCAAGAA TCCGATTGAA ATCCCAAGCA AAGAAGTTCA ACAAATTAGT GATGCTGCAA 360
AAAAGAATGG AGTTTACGTT TGCGTTTCTG TATCAGAGAA AGATAATGCC TCGCTATATT 420
TGACGCAATT GTGCTTTGAC CCGAATGGTA ATTTGATTGG CAAGCACAGG AAATTCAAGC 480
CCACTAGTAG TGAAAGAGCT GTATGGGGAG ATGGGGATGG AAGCATGGCT CCCGTATTTA 540
AAACAGAGTA TGGGAATCTT GGGGGACTCC AGTGCTGGGA ACATGCTCTC CCATTAAACA 600
TTGCGGCGAT GGGCTCATTG AACGAACAGG TACATGTTGC TTCGTGGCCA GCCTTCGTCC 660

CTAAAGGCGC AGTATCATCC AGAGTATCAT CCAGCGTCTG TGCGTCTACT AATGCGATGC 720
ATCAGATCAT TAGTCAGTTT TACGCGATCA GCAATCAGGT ATATGTAATT ATGTCAACCA 780
ATCTCGTTGG CCAAGACATG ATTGACATGA TTGGGAAAGA TGAATTTTCC AAAAACTTTC 840
TACCGCTTGG TTCTGGAAAC ACAGCGATTA TTTCTAACAC CGGTGAGATT TTGGCATCAA 900
TTCCACAAGA CGGGGAGGGA ATTGCTGTTG CAGAGATTGA CCTTAACCAA ATAATTTATG 960
GAAAGTGGTT ACTGGATCCC GCCGGTCATT ACTCTACTCC CGGCTTCTTA AGTTTGACAT 1020
TTGATCAGTC TGAACATGTA CCCGTAAAAA AAATAGGTGA GCAGACAAAC CATTTCATCT 1080
CTTATGAAGA CTTACATGAA GATAAAATGG ATATGCTAAC GATTCCGCCG AGGCGCGTAG 1140
CCACAGCGTG ATCGCCGCCT CTCGGGGCGT TCGGTTGCTG ATAGCCATCG CCTT 1194

3. Polypeptides resulting from the expression of a DNA sequence according to either of claims 1 and 2, and possessing nitrilase activity.

4. Polypeptide according to claim 3, characterized in that it comprises the sequence designated SEQ ID NO : 5 : in the attached sequence listing and given below:

Met Lys Asn Tyr Pro Thr Val Lys Val Ala Ala Val Gln Ala Ala Pro
1               5                   10                  15
Val Phe Met Asn Leu Glu Ala Thr Val Asp Lys Thr Cys Lys Leu Ile
            20                  25                  30
Ala Glu Ala Ala Ser Met Gly Ala Lys Val Ile Gly Phe Pro Glu Ala
        35                  40                  45
Phe Ile Pro Gly Tyr Pro Tyr Trp Ile Trp Thr Ser Asn Met Asp Phe
    50                  55                  60
Thr Gly Met Met Trp Ala Val Leu Phe Lys Asn Ala Ile Glu Ile Pro
65                  70                  75                  80
Ser Lys Glu Val Gln Gln Ile Ser Asp Ala Ala Lys Lys Asn Gly Val
                85                  90                  95
Tyr Val Cys Val Ser Val Ser Glu Lys Asp Asn Ala Ser Leu Tyr Leu
            100                 105                 110
Thr Gln Leu Trp Phe Asp Pro Asn Gly Asn Leu Ile Gly Lys His Arg
        115                 120                 125
Lys Phe Lys Pro Thr Ser Ser Glu Arg Ala Val Trp Gly Asp Gly Asp
    130                 135                 140
Gly Ser Met Ala Pro Val Phe Lys Thr Glu Tyr Gly Asn Leu Gly Gly
145                 150                 155                 160

Leu Gln Cys Trp Glu His Ala Leu Pro Leu Asn Ile Ala Ala Met Gly
                165                 170                 175
Ser Leu Asn Glu Gln Val His Val Ala Ser Trp Pro Ala Phe Val Pro
            180                 185                 190
Lys Gly Ala Val Ser Ser Arg Val Ser Ser Ser Val Cys Ala Ser Thr
        195                 200                 205
Asn Ala Met His Gln Ile Ile Ser Gln Phe Tyr Ala Ile Ser Asn Gln
    210                 215                 220
Val Tyr Val Ile Met Ser Thr Asn Leu Val Gly Gln Asp Met Ile Asp
225                 230                 235                 240
Met Ile Gly Lys Asp Glu Phe Ser Lys Asn Phe Leu Pro Leu Gly Ser
                245                 250                 255

5. Microorganism containing the DNA sequence (SEQ ID NO : 4 :) according to claim 1 or 2.

6. Microorganism containing the DNA sequence (SEQ ID NO : 4 :) according to claim 1 or 2 on a plasmid containing a means of selection.

7. Microorganism consisting of the *E. coli* TG1 strain containing the plasmid pXL2148, this strain being referenced G4207 and deposited in the Collection Nationale de Cultures de Micro-organismes [National Collection of Microorganism Cultures] under the No. I-1242.

8. Microorganism containing an expression cassette consisting of the DNA sequence (SEQ ID NO : 4 :) according to claim 1 or 2 under the control of signals providing for the expression of this sequence in the host microorganism.

9. Microorganism according to claim 8, characterized in that it comprises, upstream of the DNA sequence (SEQ ID NO : 4 :), a ribosome binding site and a promoter sequence which is homologous or heterologous with respect to the polypeptide produced.

10. Microorganism according to claim 9, characterized in that the promoter is chosen from the following promoters: the promoter of the tryptophan operon Ptrp of *E. coli,* the promoter of the lactose operon Plac of *E. coli;* the right-hand promoter of phage lambda P_{R}, the left-hand promoter of phage lambda P_{L}, and the strong promoters of Corynebacterium, of Comamonas or of *Pseudomonas.*

11. Microorganism according to claim 9, characterized in that the ribosome binding site is the one derived from the CII gene of phage lambda or the ones derived from *E. coli, Comamonas, Pseudomonas* or Corynebacterium genes.

12. Microorganism according to claims 8 to 11, characterized in that the expression cassette is carried by a plasmid containing a means of selection.

13. Microorganism according to claim 12, characterized in that the means of selection is a marker conferring resistance to an antibiotic.

14. Microorganism according to claims 5 to 13, characterized in that it is chosen from *E. coli, Comamonas, Corynebacterium, Brevibacterium, Rhodococcus* and *Pseudomonas* strains.

15. Microorganism according to any one of claims 5 to 14, characterized:
- in that it contains at least one proteinaceous agent that assists in the folding of the polypeptides which it synthesizes, and preferably the polypeptides according to claim 3 or claim 4, and/or the genes coding for such an agent,
- and in that this agent is present in a larger amount than that corresponding to the baseline level of the microorganism in question.

16. Microorganism according to claim 15, characterized in that the agent is the chaperone GroE of *E. coli* or its homologue of eukaryotic or prokaryotic origin.

17. Microorganism according to claim 15 or claim 16, characterized in that the genes coding for the agent are carried by the chromosome or by an extrachromosomal element (plasmid, phage), and in that they are amplified.

18. Microorganisms according to claim 17, characterized in that the genes coding for the agent are under the control of expression systems which are homologous or heterologous with respect to the said microorganism.

19. Method of enzymatic conversion of nitriles, characterized in that it consists in bringing the nitriles into contact with a polypeptide having nitrilase activity according to either of claims 3 and 4 or a host microorganism according to any one of claims 5 to 18.

20. Method according to claim 19, characterized in that the nitrile is a dinitrile of formula NC-R-CN in which R is an alkylene group having from 1 to 10 carbon atoms.

21. Method according to either of claims 19 and 20, characterized in that the nitrile is adiponitrile.

## Patentansprüche

1. DNA-Sequenz, die ein Polypeptid mit einer Nitrilaseaktivität mit der Fähigkeit zur Hydrolyse von Nitrilen in Carboxylate codiert, dadurch **gekennzeichnet**, daß sie ausgewählt ist aus:
- der DNA-Sequenz, ein Polypeptid mit einer Nitrilaseaktivität codiert, wie in der Figur 4 dargestellt ist und im beigefügten Sequenzprotokoll als SEQ ID NO: 4 bezeichnet ist,
- einem Analogon dieser Sequenz als Ergebnis der Degeneriertheit des genetischen Codes,
- einer DNA-Sequenz,die mit einer dieser Sequenzen oder einem Fragment davon hybridisiert und ein Polypeptid mit Nitrilaseaktivität codiert.

2. Rekombinante DNA-Sequenz nach Anspruch 1, dadurch **gekennzeichnet**, daß sie die folgende Nucleotidsequenz (SEQ ID NO: 4) enthält:

CTCGAGACAA AATTGGGAGA GTCGCCCCCT ATCTGCAAAA TGGAACCTCC TTTGCACATC 60
TATAAAATTT TTTGAGGAAG ACAGCAATGA AAAATTATCC TACAGTCAAG GTAGCAGCAG 120
TGCAAGCTGC TGCTGTATTT ATGAATCTAG AGGCAACAGT AGATAAAACT TGTAAGTTAA 180
TAGCAGAAGC AGCATCTATG GCGGCCAAGG TTATCGGCTT CGCAGAAGCA TTTATTCGCG 240
GCTATCCATA TTGGATTTGG ACATCAAATA TGGACTTCAC TGGAATGATG TGGGCCGTCC 300
TTTTCAAGAA TGGGATTGAA ATCCCAAGCA AAGAAGTTCA ACAAATTAGT GATGCTGCAA 360
AAAAGAATGG AGTTTAGGTT TGCGTTTCTG TATCAGAGAA AGATAATGCC TGCCTATATT 420
TGACGCAATT GTGCTTTGAC CCGAATCGTA ATTTGATTGG CAAGCACAGG AAATTCAAGC 480
CCACTAGTAG TGAAAGAGCT GTATGGGGAG ATGGGGATGG AAGCATGGCT CCCGTATTTA 540
AAACAGAGTA TGGGAATCTT GGGGGACTCC AGTGCTGGGA ACATGCTCTC CCATTAAACA 600
TTGCGGCGAT GGGCTCATTG AACGAACAGG TACATCTTGC TTCCTGGCCA GCCTTCGTCC 660
CTAAAGCGCC AGTATCATCC AGAGTATCAT CGAGCGTCTG TGCGTCTACT AATGCGATCC 720
ATCAGATCAT TAGTCAGTTT TACGCGATCA GCAATCAGCT ATATGTAATT ATGTCAACCA 780
ATCTCGTTGG CCAAGACATG ATTGACATGA TTGGGAAAGA TGAATTTTCC AAAAACTTTC 840
TACCCCTTGG TTCTGGAAAC ACAGCGATTA TTTCTAAGAC CGGTGAGATT TTGGCATCAA 900
TTCCACAAGA CGCCGAGGGA ATTGCTGTTG CAGAGATTGA CCTTAACCAA ATAATTTATG 960

GAAAGTGGTT ACTGGATCCG GCGGGTCATT ACTCTACTCG CGGCTTCTTA AGTTTGACAT 1020
TTGATCAGTC TGAACATGTA CGGGTAAAAA AAATAGGTGA GCAGACAAAC CATTTCATCT 1080
CTTATGAAGA CTTACATGAA GATAAAATGG ATATGCTAAC GATTCGCCGG AGGCGCGTAG 1140
CGACAGCGTG ATCGGCGCCT CTCGGCGCGT TCGGTTCCTG ATAGCCATCG CCTT 1194

3. Polypeptid als Ergebnis der Expression einer DNA-Sequenz nach einem der Ansprüche 1 oder 2 und mit einer Nitrilaseaktivität.

4. Polypeptid nach Anspruch 3, dadurch **gekennzeichnet**, daß es die nachstehend angegebene Sequenz mit der Bezeichnung SEQ ID NO: 5 im beigefügten Sequenzprotokoll enthält:

Met Lys Asn Tyr Pro Thr Val Lys Val Ala Ala Val Gln Ala Ala Pro
1               5                   10                  15
Val Phe Met Asn Leu Glu Ala Thr Val Asp Lys Thr Cys Lys Leu Ile
            20                  25                  30
Ala Glu Ala Ala Ser Met Gly Ala Lys Val Ile Gly Phe Pro Glu Ala
        35                  40                  45
Phe Ile Pro Gly Tyr Pro Tyr Trp Ile Trp Thr Ser Asn Met Asp Phe
    50                  55                  60
Thr Gly Met Met Trp Ala Val Leu Phe Lys Asn Ala Ile Glu Ile Pro
65                  70                  75                  80
Ser Lys Glu Val Gln Gln Ile Ser Asp Ala Ala Lys Lys Asn Gly Val
                85                  90                  95
Tyr Val Cys Val Ser Val Ser Glu Lys Asp Asn Ala Ser Leu Tyr Leu
            100                 105                 110
Thr Gln Leu Trp Phe Asp Pro Asn Gly Asn Leu Ile Gly Lys His Arg
        115                 120                 125
Lys Phe Lys Pro Thr Ser Ser Glu Arg Ala Val Trp Gly Asp Gly Asp
    130                 135                 140
Gly Ser Met Ala Pro Val Phe Lys Thr Glu Tyr Gly Asn Leu Gly Gly
145                 150                 155                 160
Leu Gln Cys Trp Glu His Ala Leu Pro Leu Asn Ile Ala Ala Met Gly
                165                 170                 175
Ser Leu Asn Glu Gln Val His Val Ala Ser Trp Pro Ala Phe Val Pro
            180                 185                 190

Lys Gly Ala Val Ser Ser Arg Val Ser Ser Ser Val Cys Ala Ser Thr
        195                 200                 205
Asn Ala Met His Gln Ile Ile Ser Gln Phe Tyr Ala Ile Ser Asn Gln
    210                 215                 220
Val Tyr Val Ile Met Ser Thr Asn Leu Val Gly Gln Asp Met Ile Asp
225                 230                 235                 240
Met Ile Gly Lys Asp Glu Phe Ser Lys Asn Phe Leu Pro Leu Gly Ser
                245                 250                 255

Gly Asn Thr Ala Ile Ile Ser Asn Thr Gly Glu Ile Leu Ala Ser Ile
            260                 265                 270
Pro Gln Asp Ala Glu Gly Ile Ala Val Ala Glu Ile Asp Leu Asn Gln
        275                 280                 285
Ile Ile Tyr Gly Lys Trp Leu Leu Asp Pro Ala Gly His Tyr Ser Thr
    290                 295                 300
Pro Gly Phe Leu Ser Leu Thr Phe Asp Gln Ser Glu His Val Pro Val
305                 310                 315                 320
Lys Lys Ile Gly Glu Gln Thr Asn His Phe Ile Ser Tyr Glu Asp Leu
                325                 330                 335
His Glu Asp Lys Met Asp Met Leu Thr Ile Pro Pro Arg Arg Val Ala
            340                 345                 350
Thr Ala

5. Mikroorganismus, enthaltend die DNA-Sequenz (SEQ ID NO: 4) nach den Ansprüchen 1 oder 2.

6. Mikroorganismus, enthaltend die DNA-Sequenz (SEQ ID NO: 4) nach den Ansprüchen 1 oder 2 in einem Plasmid mit einem Selektionsmarker.

7. Mikroorganismus, bestehend aus dem das Plasmid pXL2148 enthaltenden *E. coli*-Stamm TG1, bezeichnet als G4207 und hinterlegt bei der Collection Nationale de Cultures de Microorganismes unter der Hinterlegungsnummer I-1242.

8. Mikroorganismus, enthaltend eine Expressionskassette aus der DNA-Sequenz (SEQ ID No: 4) nach Anspruch 1 oder 2 unter der Steuerung von Signalen, die die Expression dieser Sequenz in dem Wirtsmikroorganismus gewährleisten.

9. Mikroorganismus nach Anspruch 8, dadurch **gekennzeichnet**, daß er stromaufwärts der DNA-Sequenz (SEQ ID NO: 4) eine Ribosomenbindungsstelle und eine für das produzierte Polypeptid homologe oder heterologe Promotorsequenz umfaßt.

10. Mikroorganismus nach Anspruch 9, dadurch **gekennzeichnet**, daß der Promotor aus den folgenden Promotoren ausgewählt ist: Promotor des Tryptophanoperons Ptrp von *E. coli,* Promotor des Lactoseoperons Plac von *E. coli,* rechter Promotor des Phagen Lambda P_{R}, linker Promotor des Phagen Lambda P_{L}, und die starken Promotoren von *Corynebacterium, Comamonas* oder *Pseudomonas.*

11. Mikroorganismus nach Anspruch 9, dadurch **gekennzeichnet**, daß die Ribosomenbindungsstelle diejenige ist, die von dem Gen CII des Phagen Lambda abgeleitet ist, oder diejenigen sind, die von den Genen von E. coli, *Corynebacterium, Comamonas* oder *Pseudomonas* abgeleitet sind.

12. Mikroorganismus nach den Ansprüchen 8 bis 11, dadurch **gekennzeichnet**, daß die Expressionskassette auf einem Plasmid mit einem Selektionsmarker vorhanden ist.

13. Mikroorganismus nach Anspruch 12, dadurch **gekennzeichnet**, daß der Selektionsmarker ein Marker ist, der Antibiotikaresistenz verleiht.

14. Mikroorganismus nach den Ansprüchen 5 bis 13, dadurch **gekennzeichnet**, daß er aus den Stämmen *E. coli, Comamonas, Corynebacterium, Brevibacterium, Rhodococcus, Pseudomonas* ausgewählt ist.

15. Mikroorganismus nach einem der Ansprüche 5 bis 14, dadurch **gekennzeichnet**, daß
- er mindestens ein proteinisches Hilfsmittel zur Faltung von Polypeptiden, die er synthetisiert, und bevorzugt der Polypeptide nach Anspruch 3 oder 4, und/oder die dieses Mittel codierenden Gene enthält,
- und dieses Mittel in einer Menge vorhanden ist, die größer ist als die, die der Grundmenge des betrachteten Mikroorganismus entspricht.

16. Mikroorganismus nach Anspruch 15, dadurch **gekennzeichnet**, daß das Mittel das Chaperon GroE von *E. coli* oder ein Homologes davon mit eukaryotischem oder prokaryotischem Ursprung ist.

17. Mikroorganismus nach Anspruch 15 oder 16, dadurch **gekennzeichnet**, daß die Gene, die das Mittel codieren, auf einem Chromosom oder einem extrachromosomalen Element (Plasmid, Phage) vorhanden sind, und daß sie amplifiziert sind.

18. Mikroorganismen nach Anspruch 17, dadurch **gekennzeichnet**, daß die Gene, die das Mittel codieren, unter der Steuerung von für den Mikroorganismus homologen oder heterologen Expressionssystemen stehen.

19. Verfahren zur enzymatischen Transformation von Nitrilen, dadurch **gekennzeichnet**, daß man die Nitrile mit einem Polypeptid mit einer Nitrilaseaktivität nach einem der Ansprüche 3 oder 4 oder einem Wirtsmikroorganismus nach einem der Ansprüche 5 bis 18 in Kontakt bringt.

20. Verfahren nach Anspruch 19, dadurch **gekennzeichnet**, daß das Nitril ein Dinitril der Formel NC-R-CN ist, worin R ein Alkylenrest mit 1 bis 10 Kohlenstoffatomen ist.

21. Verfahren nach einem der Ansprüche 19 oder 20, dadurch **gekennzeichnet**, daß das Nitril Adiponitril ist.
